# EUROPEAN PATENT APPLICATION

(11) **EP 4 763 090 A1**
(43) Date of publication of application: **24.06.2026**
(21) Application number: 24220744.7
(22) Date of filing: 17.12.2024
(51) Int. Cl.: A61B 6/03, A61B 6/04, A61B 6/58

(54) **CALIBRATING OF A MEDICAL IMAGING SYSTEM**

(71) Applicant: Koninklijke Philips N.V., 5656 AE Eindhoven (NL)
(72) Inventor: KRUEGER, Sascha, Eindhoven (NL); RUTJES, Bart, Eindhoven (NL); KOK, Annette, 5656AG Eindhoven (NL); SENEGAS, Julien Thomas, Eindhoven (NL); WIRTZ, Daniel, Eindhoven (NL); WIEMKER, Rafael, Eindhoven (NL); VAN EE, Raymond, Eindhoven (NL)
(74) Representative: Philips Intellectual Property & Standards

(57) **Abstract**

Disclosed herein is a method for calibrating a medical imaging system (102). The method comprises determining a first relative position of an imaging phantom (118) on a movable patient table (120) relative to the patient table (120) using first photosensor imaging data (414) of the patient table (120) in an initial first position. A second relative position of a second position of the patient table (120) with the imaging phantom (118) arranged within an imaging zone (157) of the medical imaging system (102) relative to a table reference position is determined. A third relative position of the imaging phantom (118) relative to an imaging reference position of the medical imaging system (102) is determined using medical imaging data (416). A relative position of the imaging reference position to the table reference position is determined using the determined first, second, and third relative position.

## Description

### FIELD OF THE INVENTION

The invention relates to medical imaging, in particular to a method for calibrating a medical imaging system. The calibrating comprises a determining of a relative position of an imaging reference position of the medical imaging system relative to a table reference position of a movable patient table using a photosensor imaging system. Furthermore, the invention relates to a computer program for calibrating the medical imaging system and a medical system comprising a computational system for calibrating the medical imaging system.

### BACKGROUND OF THE INVENTION

For a supervision of medical imaging systems, like magnetic resonance imaging systems, computed tomography systems, or X-ray imaging systems, photosensor imaging systems may be used. In order to be able to determine positions of target structures, like a patient and/or components of the medical imaging system, within photosensor imaging data acquired using such a photosensor imaging system, a calibration of the medical imaging system may be necessary.

### SUMMARY OF THE INVENTION

The invention provides for a method for calibrating a medical imaging system in the independent claims. The invention further provides for a computer program as well as for a medical system comprising a computational system for calibrating the medical imaging system in the independent claims.

In one aspect, a method for calibrating a medical imaging system is disclosed. The calibrating comprises by a computational system a determining of a relative position of an imaging reference position of the medical imaging system relative to a table reference position of a movable patient table using a photosensor imaging system. The photosensor imaging system is, e.g., configured for a supervision of the medical imaging system with the patient table. The method comprises receiving first photosensor imaging data acquired using the photosensor imaging system. The first photosensor imaging data comprises photosensor imaging data of the patient table in an initial first position with an imaging phantom arranged on the patient table. The method comprises determining a first relative position of the imaging phantom relative to the patient table using the first photosensor imaging data. The method comprises determining a second relative position of a second position of the patient table relative to the table reference position. In the second position of the patient table the imaging phantom is arranged within an imaging zone of the medical imaging system.

The method comprises receiving medical imaging data of the imaging phantom arranged within the imaging zone, which is acquired by the medical imaging system. The method comprises determining a third relative position of the imaging phantom relative to the imaging reference position of the medical imaging system using the medical imaging data. The method comprises determining the relative position of the imaging reference position of the medical imaging system relative to the table reference position of the patient table using the determined first, second, and third relative position.

In another aspect, a computer program for calibrating a medical imaging system is disclosed. The computer program comprises machine executable instructions for an execution by a computational system. Execution of the machine executable instructions causes the computational system to perform a method for calibrating the medical imaging system. The calibrating comprises by the computational system a determining of a relative position of an imaging reference position of the medical imaging system relative to a table reference position of a movable patient table using a photosensor imaging system. The photosensor imaging system is, e.g., configured for a supervision of the medical imaging system with the patient table. The method comprises receiving first photosensor imaging data acquired using the photosensor imaging system. The first photosensor imaging data comprises photosensor imaging data of the patient table in an initial first position with an imaging phantom arranged on the patient table. The method comprises determining a first relative position of the imaging phantom relative to the patient table using the first photosensor imaging data. The method comprises determining a second relative position of a second position of the patient table relative to the table reference position. In the second position of the patient table the imaging phantom is arranged within an imaging zone of the medical imaging system.

The method comprises receiving medical imaging data of the imaging phantom arranged within the imaging zone, which is acquired by the medical imaging system. The method comprises determining a third relative position of the imaging phantom relative to the imaging reference position of the medical imaging system using the medical imaging data. The method comprises determining the relative position of the imaging reference position of the medical imaging system relative to the table reference position of the patient table using the determined first, second, and third relative position.

In another aspect, a medical system comprising a computational system for calibrating a medical imaging system is disclosed. The computational system comprises a memory storing machine executable instructions. Execution of the machine executable instructions causes the computational system to perform a method for calibrating the medical imaging system. The calibrating comprises by the computational system a determining of a relative position of an imaging reference position of the medical imaging system relative to a table reference position of a movable patient table using a photosensor imaging system. The photosensor imaging system is, e.g., configured for a supervision of the medical imaging system with the patient table. The method comprises receiving first photosensor imaging data acquired using the photosensor imaging system. The first photosensor imaging data comprises photosensor imaging data of the patient table in an initial first position with an imaging phantom arranged on the patient table. The method comprises determining a first relative position of the imaging phantom relative to the patient table using the first photosensor imaging data. The method comprises determining a second relative position of a second position of the patient table relative to the table reference position. In the second position of the patient table the imaging phantom is arranged within an imaging zone of the medical imaging system.

The method comprises receiving medical imaging data of the imaging phantom arranged within the imaging zone, which is acquired by the medical imaging system. The method comprises determining a third relative position of the imaging phantom relative to the imaging reference position of the medical imaging system using the medical imaging data. The method comprises determining the relative position of the imaging reference position of the medical imaging system relative to the table reference position of the patient table using the determined first, second, and third relative position.

### BRIEF DESCRIPTION OF THE DRAWINGS

In the following preferred embodiments of the invention will be described, by way of example only, and with reference to the drawings in which:
Fig. 1 shows an exemplary method for calibrating a medical imaging system using a photosensor imaging system;
Fig. 2 shows an exemplary method for calibrating a medical imaging system using a photosensor imaging system;
Fig. 3 shows an exemplary method for controlling a movement of the movable patient table;
Fig. 4 shows an exemplary method for calibrating a medical imaging system using a photosensor imaging system;
Fig. 5 shows an exemplary method for training a keypoint elements detection module;
Fig. 6 shows an exemplary method for training a phantom position detection module;
Fig. 7 shows a method for selecting photosensor imaging data to be used for a calibration;
Fig. 8 illustrates exemplary photosensor imaging training data for training a keypoint detection module to detect keypoint elements of a patient table;
Fig. 9 illustrates exemplary photosensor imaging training data for training a keypoint detection module to detect keypoint elements of an imaging phantom;
Fig. 10 illustrates an exemplary detection of keypoint elements in photosensor imaging data of a patient table with an imaging phantom;
Fig. 11 illustrates an exemplary detection of keypoint elements in photosensor imaging data of a patient table;
Fig. 12 illustrates exemplary photosensor imaging data of a patient table with an imaging phantom;
Fig. 13 illustrates exemplary photosensor imaging data of a patient table with an imaging phantom;
Fig. 14 illustrates an exemplary computational system for calibrating a medical imaging system using a photosensor imaging system; and
Fig. 15 illustrates an exemplary medical system comprising a computational system for calibrating a medical imaging system using a photosensor imaging system.

### DETAILED DESCRIPTION OF EMBODIMENTS

Like-numbered elements in these figures are either equivalent elements or perform the same function. Elements which have been discussed previously will not necessarily be discussed in later figures if the function is equivalent.

For example, a method for calibrating a medical imaging system is provided. The calibrating comprises by a computational system a determining of a relative position of an imaging reference position of the medical imaging system relative to a table reference position of a movable patient table using a photosensor imaging system. The photosensor imaging system is, e.g., configured for a supervision of the medical imaging system with the patient table. The method comprises receiving first photosensor imaging data acquired using the photosensor imaging system. The first photosensor imaging data comprises photosensor imaging data of the patient table in an initial first position with an imaging phantom arranged on the patient table. A first relative position of the imaging phantom relative to the patient table is determined using the first photosensor imaging data. A second relative position of a second position of the patient table relative to the table reference position is determined. In the second position of the patient table the imaging phantom is arranged within an imaging zone of the medical imaging system.

Medical imaging data of the imaging phantom arranged within the imaging zone, which is acquired by the medical imaging system, is received. A third relative position of the imaging phantom relative to the imaging reference position of the medical imaging system is determined using the medical imaging data. The relative position of the imaging reference position of the medical imaging system relative to the table reference position of the patient table is determined using the determined first, second, and third relative position.

Examples may enable a determining relative position of the imaging reference position of the medical imaging system relative to the table reference position of the patient table. Thus, positions of the table defined with respect to the table reference position, e.g., a home position of the patient table, may be related to the imaging reference position. With the relative position between imaging reference position and table reference position known, e.g., a relative position of the patient table and the imaging reference position for any current position of the patient table may easily be determined by determine a relative position of the patient table and the table reference position as well as using the relative position between imaging reference position and table reference position. With a relative position of the patient table and the imaging reference position for any current position of the patient table known, e.g., a moving of the patient table from the current position to the imaging reference position or to a target position relative to the imaging reference position may be controlled.

The first relative position is a relative position of the imaging phantom relative to the patient table. The second relative position is a relative position of the patient table relative to the table reference position with the imaging phantom arranged in the imaging zone of the medical imaging system. Thus, a combination of the first and second relative position may describe a relative position of the imaging phantom to the table reference position, when the imaging phantom is arranged in the imaging zone of the medical imaging system.

The third relative position is a relative position of the imaging phantom relative to the imaging reference position of the medical imaging system. Thus, a combination of the first, second, and third relative position describes the relative position of the imaging reference position of the medical imaging system relative to the table reference position of the patient table, i.e., the relative position of the imaging reference position to the imaging phantom and from the imaging phantom to the table reference position.

For the determining of the first relative position, first photosensor imaging data may be used. In this first photosensor imaging data, e.g., the patient table and the imaging phantom may be detected and their spatial relation determined.

For the determining of the second relative position, e.g., second photosensor imaging data may be used. With the first and second photosensor imaging data a relative position of the second position of the patient table and the initial first position may be determined. For example, the initial first position may be the table reference position. Thus, the relative position of the second position of the patient table and the initial first position may correspond to a relative position of the second position of the patient table and the table reference position. For example, the initial first position may be different from the table reference position. In this case, e.g., a relative position of the initial first position of the patient table relative to the table reference position may be determined in addition. For example, photosensor imaging data of the patient table arranged at the table reference position may be used for this purpose.

For the determining of the second relative position, e.g., sensor data of a drive device for moving the patient table may be used. The sensor data of the drive device may be descriptive of a moving of the patient table from the initial first position to the second position. Thus, the sensor data may be descriptive of a relative position of the second position of the patient table and the initial first position. Furthermore, sensor data of the drive device descriptive of a moving of the patient table from the table reference position to the initial first position of the patient table may be provided, in case the initial first position is not identical with the table reference position.

When moving the patient table with the imaging phantom from the initial first position to the second position with the imaging phantom arranged within an imaging zone of the medical imaging system, the imaging phantom may, e.g., reach an intermediate position, at which the imaging phantom is aligned with a light visor cross of the medical imaging device. The light visor cross may define a physical reference position relative to a hardware of the medical imaging system, e.g., to a gantry of the medical imaging system. For example, photosensor imaging data acquired by the photosensor imaging system may be used for controlling a movement of the patient table from the initial first position to the intermediate position, at which the imaging phantom is aligned with the light visor cross. For example, the initial first position is identical with the respective position and the imaging phantom is arranged on the patient table, such that it is aligned with the light visor cross. For example, the initial first position is identical with the position of the patient table, in which the imaging phantom is aligned with the light visor cross.

Examples may, e.g., enable an automatic calibration of a transformation from the table reference position, e.g., a home position of the patient table, to the imaging reference position, e.g., an imaging iso-center of the medical imaging system, for the photosensor imaging system. The photosensor imaging system may, e.g., provide a sensor system for a supervision of an imaging workflow executed using the medical imaging system.

Examples may enable to determine precisely a position of the patient table relative to the imaging reference position of the medical imaging system. Thus, the relative position of the patient table within the medical imaging system is known and may, e.g., be used for controlling a positioning of the patient table and/or a patient arranged thereon relative to the imaging reference position. Using the patient table of the medical imaging system for the calibration may have the beneficial effect that the patient table can in general be moved in three dimensions with locations of keypoint elements of the patient table accurately determinable using the photosensor imaging system.

The patient table may, e.g., be configured to be moved, e.g., in one, two, or three dimensions, relative to the rest of the medical imaging system, i.e., the imaging reference position.

The photosensor imaging system may, e.g., be used for an automated positioning of a patient arranged on the patient table at the imaging reference position or at a target position relative to the imaging reference position. The photosensor imaging system may further, e.g., be used for guiding and automating a patient examination setup where needed and, e.g., to detect non-ideal and/or unsafe system uses or setup configurations. For advanced use-cases of such a sensor system a calibration of the photosensor imaging system with the medical imaging system, e.g., a magnetic resonance imaging system, may be executed.

The calibration may, e.g., comprise a calibration of the medical imaging system. An aim of the calibration of the medical imaging system may, e.g., be establishing a calibration of a table reference position, e.g., a home position, with respect to an imaging reference position of the medical imaging system, e.g., an imaging iso-center, without adding additional work steps for a service technician. For this purpose, e.g., a relative position of the imaging reference position of the medical imaging system relative to a table reference position of the patient table, e.g., a home position of the patient table, may be determined. This relative position is descriptive of a spatial relation between the imaging reference position of the medical imaging system and the table reference position of the patient table. With the spatial relation between the imaging reference position of the medical imaging system relative and the table reference position of the patient table known, e.g., table positions may be determined with respect to the imaging reference position. For example, the table positions may be determined relative to the table reference position, for which the spatial relation to the imaging reference position of the medical imaging system is known. Thus, also the spatial relation of the table positions relative to the imaging reference position of the medical imaging system may be determined.

Examples may enable an implementation of the calibration in a tomographic imaging modality, which in general is non-trivial especially, when a number of work steps for field service should be kept to a minimum for cost reasons.

A patient setup, e.g., a preparation, in a medical imaging system is a time-consuming task, which in general requires trained personnel. The complexity of this task may draw attention away from the patient even for trained and skilled operators. Trying to execute this task in an effective and efficient way may result in increased stress levels for the operators. Thus, there is a danger that this situation may result in a limitation of consistency of examination quality, that it may impair patient's and/or operator's experience, as well as that a risk of errors including harm to the patient may arise.

Photosensor imaging systems may be used to address these issues by providing assistance in the patient setup where needed. Furthermore, assistance may be provided in detecting non-ideal and/or unsafe system uses as well as non-ideal and/or unsafe setup configurations.

The assistance may, e.g., rely on detecting the patient body and anatomy, as well as relevant parts of equipment of the medical imaging system based on photosensor imaging data acquired using the photosensor imaging system. Therefore, a high precision and robustness of the detection may be required.

The photosensor imaging system may, e.g., comprise one or more imaging photosensors. The photosensor imaging system may be arranged relative to the medical imaging system with the patient table, such that the patient table is contained in the field of view of the photosensor imaging system. For example, the photosensor imaging system may be arranged above the patient table looking from above onto the patient table. For example, the photosensor imaging system may be arranged above the patient table a home position of the patient table.

In case of a plurality of imaging photosensors with differing fields of view being comprised by the photosensor imaging system, the imaging photosensors may be arranged relative to the medical imaging system with the patient table, such that the patient table is contained in the fields of view of the imaging photosensors of the plurality of imaging photosensors.

The photosensor imaging system may, e.g., comprise a plurality of imaging photosensors. An imaging photosensor refers to a sensor, which is configured for detecting photons for acquiring photosensor imaging data. The photosensor imaging data comprise imaging data determined using photons detected by the photosensor. The photosensor imaging data may, e.g., be provided in the form of one or more images. The imaging photosensors may, e.g., be configured for detecting photons with energies, i.e., wavelengths, within the visible electromagnetic spectrum. The imaging photosensors may, e.g., comprise one or more RGB-sensors, i.e., imaging photosensors configured to acquire photosensor imaging data comprising red (R), green (G), and blue (B) light. The imaging photosensors may, e.g., comprise one or more depth sensors, i.e., imaging photosensors configured to determine distances of objects from the sensors, e.g., using triangulation. The imaging photosensors may, e.g., be configured for detecting photons with energies beyond the visible electromagnetic spectrum. The imaging photosensors may, e.g., be configured for detecting photons with energies i.e., wavelengths, in the near-infrared range, in the infrared range and/or other ranges beyond the visible electromagnetic spectrum. The imaging photosensors may, e.g., comprise one or more thermal sensors, i.e., imaging photosensors configured to acquire photosensor imaging data in the infrared (IR) range. For example, thermal sensors may acquire photosensor imaging data of photons with wavelengths in the range from 1 µm to 15 µm, e.g., 10 µm at room temperature.

The calibration, i.e., the precise determination of the relative position of the imaging reference position to the table reference position may, e.g., be used for executing tasks which automatically compute imaging parameters, such as ideal off centers, an angulation, a field of view and/or an extent of scans.

Examples enable an establishing of a spatial relation between the table reference position and the imaging reference position of the medical imaging system, e.g., an imaging iso-center. This may, e.g., provide a simple and accurate means to calibrate a transformation between a first coordinate system, which is a table coordinate system defined relative to the table reference position, and a second coordinate system, which is an imaging coordinate system defined relative to imaging reference position. Furthermore, a reduction of field service errors as well as a reduction of system maintenance and product development costs may be enabled.

A calibration of a medical imaging system itself, which is to be installed, may, e.g., comprise a step of establishing a position defined, e.g., by a light visor, or generally speaking a user-facing reference position of the medical imaging system with respect to the imaging reference position of the medical imaging system, e.g., an imaging iso-center of the medical imaging system. To this end, e.g., a dedicated imaging phantom may be placed in a stable position on the patient table, marked with the light visor, and then moved close to the imaging reference position, e.g., the iso-center. The dedicated imaging phantom may, e.g., be a blue sphere liquid phantom with holder. The imaging phantom may be moved only close to the iso-center, since the calibration is still missing, and the missing calibration may prevent a precise targeted positioning of imaging phantom in the iso-center. Medical imaging data of the imaging phantom may be acquired and a deviation of the phantom with respect to the true iso-center may be determined using the medical imaging data. This deviation may, e.g., be added to a table travel distance from the position identified by the light visor to obtain a calibrated travel to scan (TTS) distance. Thus, e.g., an iso-centering via a light visor may be calibrated.

For a full calibration of the medical imaging system with the photosensor, additionally, e.g., a transformation dₕ from the table reference position, e.g., a table home position, to the position defined by the light visor, if available, or a transformation dᵢ from the table reference position to the imaging reference position, e.g., the iso-center, may be used. The home position may, e.g., be a predefined position of the patient table in a main patient setup preparation area. Examples may enable a determining of these transformations dₕ and/or dᵢ, even though dₕ and dᵢ may vary per system installation due to allowed tolerances. This calibration step, which determines relative position of the imaging reference position of the medical imaging system relative to the table reference position of the patient table, may be mandatory, when using a photosensor imaging system, to enable derivation of parameters in the imaging coordinate system, e.g., in an imaging iso-center calculation, from detections in the photosensor imaging system and/or table coordinate system.

Examples may in principle, e.g., be compatible with all calibration steps that involve a placement of a dedicated imaging phantom, imaging thereof, i.e., acquiring medical imaging data of the dedicated imaging phantom, and determining geometric properties of the scanner, i.e., medical imaging system, from the acquired medical imaging data.

It may be emphasized that a usage of a light visor is compatible with, but not required for executing examples.

Examples may, e.g., provide an integrated, simple and/or accurate method to intrinsically establish the aforementioned calibration of the medical imaging system, e.g., during an already existing calibration routine of the medical imaging system. The intrinsic establishing may, e.g., comprise the following steps:
1. Receiving photosensor imaging data of the patient table with the imaging phantom in some table position, i.e., first photosensor imaging data.
2. Detecting the imaging phantom in the received photosensor imaging data and calculating a phantom reference point in 3D.

For an arbitrary imaging phantom with known geometry, e.g., a projected volumetric reference point of the imaging phantom may be detected and using known lens parameters of the photosensor imaging system, a ray angle of the detected photons may be determined. A traverse length of this ray from the phantom reference point to a phantom surface of the imaging phantom may, e.g., be computed. This traverse length may, e.g., be added to a depth value of a depth sensor comprised by the photosensor imaging system to get a correct depth value.

Alternatively, multiple detections of the projected volumetric reference point from different relative positions of the photosensor imaging system and the imaging phantom may be used to triangulate a 3D position. This may, e.g., be accomplished using a stereo camera setup for the photosensor imaging system or using table movements.

3. Detecting a table center and computing a 3D coordinate of the detected table center in the table coordinate system. This 3D coordinate, e.g., equals (0,0,0), if the patient table is arranged exactly in the table reference position. This may correspond to a determining of a fourth relative position of the first position of the patient table relative to the table reference position.

4. Determining a transformation dₚ of the phantom reference point with respect to the detected table center. This may correspond to determining a first relative position of the imaging phantom relative to the patient table using the first photosensor imaging data.

5. Selecting a plane to scan, e.g., using a laser or the photosensor imaging system, and travelling to this scan plane using a preconfigured average configuration of table-scanner-geometry. The position reached by this travelling may, e.g., correspond to the second position of the patient table.

6. Receiving photosensor imaging data from the photosensor imaging system on a calibration scan start event. The received photosensor imaging data may, e.g., be the second photosensor imaging data acquired using the photosensor imaging system, which comprises photosensor imaging data of the patient table moved to the second position.

7. Extracting remaining detectible table keypoints, which are, e.g., still outside the bore, from the photosensor imaging data.

8. Determining a transformation from the patient table in imaging position, i.e., second position, to the keypoint coordinates with the patient table in the table reference position, e.g., the table home position, and deriving a transformation dₕₛ. This may correspond to determining the second relative position of the second position of the patient table relative to the table reference position. Data for the table reference position may, e.g., be stored in the system. This data may, e.g., exist from a previous calibration step. For example, a weight induced bending of the patient table may be taken into account. For this purpose, e.g., a table bending model may be used, which is e.g. a function of weight and position of a center of mass of a weight arranged on the patient table.

9. Receiving medical imaging data, e.g., executing a scanner calibration scan, and obtaining an offset d of the phantom reference point of imaging phantom from the imaging reference point, e.g., the iso-center, at the scan position. Since the medical imaging system is not yet calibrated, the imaging phantom in the scan position may in general not be arranged exactly centered at the iso-center. This may correspond to determining a third relative position of the imaging phantom relative to the imaging reference position of the medical imaging system, e.g., the imaging iso-center, using the medical imaging data. For example, an automatic detection of the phantom reference point of the imaging phantom from the medical imaging data, e.g., from a scanner calibration image, may be executed.

10. Obtaining the transformation dᵢ from the table reference position to the imaging reference position, e.g., the iso-center. The transformation dᵢ may, e.g., be determined by the relation dᵢ = dₕₛ * dₚ * d. This may correspond to determining the relative position of the imaging reference position of the medical imaging system, e.g., the imaging iso-center, relative to the table reference position of the patient table, e.g., the home position of the patient table, using the determined first, second, and third relative position.

Examples may have the beneficial effect that the patient table may be anywhere, and the imaging phantom may be placed anywhere and/or inaccurately on the patient table. Both may, e.g., be detected using photosensor imaging data acquired using the photosensor imaging system. For a CT system, e.g., the patient table may not need to be detected using photosensor imaging data, if its positions relative to the table coordinate system, i.e., the table reference position is known. The position may, e.g., be known from sensor data of a drive device used for moving the patient table.

Example may, e.g., be configured to also cover deflection and/or bending effects of the patient table. For example, in a magnetic resonance imaging system a table guide may give the patient table a bump and/or may tilt it slightly into one direction, e.g., the right direction. In a CT system, trajectories may, e.g., differ from sensor readings, e.g., if bearings are not sufficiently accurate.

Examples may, e.g., make use of the patient table's absolute positions, e.g., longitudinal position and height, as well as relative displacements, e.g., longitudinal and/or vertical displacements, measured and provided by the medical imaging system itself, rather than measuring a table transformation from the table reference position using the photosensor imaging system. Absolute position and relative displacement may, e.g., be measured by internal sensors of the medical imaging system, e.g., by sensors of a drive device configured for moving the patient table, and logged internally every time the table moves. The rest of the method may, e.g., stay the same.

In this case, an example may, e.g., comprise:
1. Detecting using the photosensor imaging system a position in 3D of a phantom reference point with respect to the table coordinate system for a first table position. This may, e.g., correspond to determining a first relative position of the imaging phantom relative to the patient table using the first photosensor imaging data. For example, furthermore a fourth relative position of the first position of the patient table relative to the table reference position may be determined, in order to determine a relative position of the imaging phantom relative to the table reference position.
2. Moving the phantom to or near to the imaging reference position, e.g., an imaging iso-center of the medical imaging system. This may be the second table position. Relative longitudinal and vertical displacements of the patient table between the first position and second table positions are determined and recorded.
3. Receiving medical imaging data acquired, e.g., by scanner calibration scan, and obtaining an offset d of the phantom reference point of the imaging phantom from the imaging reference point. This may correspond to determining the third relative position of the imaging phantom relative to the imaging reference position of the medical imaging system using the medical imaging data.
4. Computing from the 3D coordinates obtained in step 1), the table transformation provided in step 2), and the off-center measured in step 3), the second relative position of the second position of the patient table relative to the table reference position.

Examples may provide means to secure and maintain optimal system performance by minimal service/maintenance effort.

A module, e.g., a keypoint detection module, for detecting the calibration phantom and the patient table may comprise one or more neural network. For example, the module may comprise two neural networks used to detect the calibration phantom and the patient table respectively. For detecting the imaging phantom, a detection neural network may be used, which provides a module for robust detection of a phantom reference point, e.g., a center of the imaging phantom. Such reference points may, e.g., be accurately described for calibration phantoms, which are constructed with high precision. Since a 3D geometry of an imaging phantom is thus in general precisely known, retrieving the 3D coordinates from the received photosensor imaging data may be straightforward. This may, e.g., be achieved with help of a photosensor configured as a depth sensor, with multiple imaging photosensors using stereoscopic techniques, and/or using a direct triangulation of the phantom reference point.

An imaging phantom may, e.g., be used with a holder, which may, e.g., consist of or contain reflective plastic and/or other materials, or the imaging phantom may, e.g., contain such materials itself. Also, an imaging phantom may, e.g., optically degrade with time and change its appearance. For example, air bubbles may form inside the imaging phantom. All these effects may significantly impact a variety of optical appearances of the imaging phantom and may make a usage of detection methods more difficult.

These property variations may, e.g., be included into a development of training data for the detecting modules, like the keypoint detection module and/or the phantom position detection module, e.g., via simulation.

For example, method further comprises receiving second photosensor imaging data acquired using the photosensor imaging system. The second photosensor imaging data comprises photosensor imaging data of the patient table moved to the second position. The second photosensor imaging data is used for the determining of the second relative position of the second position of the patient table relative to the table reference position.

Examples may enable a determining of the second relative position using photosensor imaging data.

For example, the initial first position differs from the table reference position. The method further comprises determining a fourth relative position of the first position of the patient table relative to the table reference position. A fifth relative position of the second position of the patient table relative to the first position of the patient table is determined using the second photosensor imaging data. The fourth and fifth relative position are used for determining the second relative position.

For example, the initial first position is identical with the table reference position. In this case, e.g., only the fifth relative position of the second position of the patient table relative to the first position of the patient table may be determined using the second photosensor imaging data. The fifth relative position are used for determining the second relative position.

For example, the imaging reference position is an imaging iso-center of the medical imaging system.

For example, the table reference position is a default home position of the patient table.

For example, the first relative position of the imaging phantom is a relative position of a center of the imaging phantom. For example, the first relative position of the imaging phantom is determined relative to a center of the patient table.

For example, the computational system comprises a keypoint detection module configured for detecting as output one or more keypoint elements of the patient table within the first photosensor imaging data in response to receiving the first photosensor imaging data as input. The keypoint detection module is further configured for detecting as output one or more keypoint elements of the imaging phantom within the first photosensor imaging data received as input.

The method further comprises determining first keypoint elements of the patient table within the first photosensor imaging data using the keypoint detection module. Keypoint elements of the imaging phantom within the first photosensor imaging data using the keypoint detection module are determined. The determined first keypoint elements of the patient table and the determined keypoint elements of the imaging phantom are used for the determining of the first relative position.

For example, the keypoint detection module may be used for detecting specific keypoint elements of a patient table. For example, the keypoint detection module may be used for detecting specific keypoint elements of an imaging phantom. Such a keypoint detection module may be implemented in the form of a machine learning module. For example, the keypoint detection module may comprise a neural network. For example, the keypoint detection module may use a fully convolutional neural network for detecting keypoint elements. For example, the keypoint detection module may use a fully convolutional neural network with a U-Net architecture for detecting keypoint elements. The U-Net architecture is described in Olaf Ronneberger, "U-Net: Convolutional Networks for Biomedical Image Segmentation" (https://arxiv.org/abs/1505.04597). For example, a ResNet, i.e. Residual Neural Network as described in Kaiming He et al., "Deep Residual Learning for Image Recognition" (https://arxiv.org/abs/1512.03385), may be used by the keypoint detection module for detecting keypoint elements.

For example, the keypoint detection module may be configured to determine confidence levels for the detections of the keypoint elements within the photosensor imaging data. For example, the keypoint detection module may be configured for assigning confidence levels for the detections to the detected keypoint elements. The keypoint detection module may, e.g., during a training phase, be trained for assigning confidence levels to the detections to the keypoint elements detected within the training data. These confidence levels determined using the keypoint detection module may, e.g., be estimations trained using a loss function quantifying a deviation of the detections of the keypoint elements from a ground truth for the keypoint elements provided by the photosensor imaging training data. The confidence levels may, e.g., be functions of an output of the keypoint detection module. A maximum, minimum, mean, or integral of the output of the detection module may be examples of quantities, which may be used to derive a confidence metric within a training distribution provided by a plurality of photosensor imaging training data. Out-of-distribution confidence values may, e.g., be derived using a temporal stability of an output signal of the keypoint detection module, a stability against perturbances of the output signal, like noise etc., or other spatial and/or temporal properties of the output signal provided by the keypoint detection module as a response to receiving photosensor imaging data as input.

For example, a three-dimensional digital model of the patient table, e.g., a CAD model of the patient table may be provided for generating synthetic training data with defined keypoint elements and labels identifying the keypoint elements using a simulation pipeline. For example, a three-dimensional digital model of the imaging phantom, e.g., a CAD model of the imaging phantom may be provided for generating synthetic training data with defined keypoint elements and labels identifying the keypoint elements using a simulation pipeline. With the resulting synthetic training data, the keypoint detection module, e.g., a neural network comprised by keypoint detection module, is trained to detect the keypoint elements in photosensor imaging data, in particular in photosensor imaging data, i.e., in a real imaging environment. For example, a fully convolutional U-net architecture may be used to encode probability maps of keypoint elements, e.g., two-dimensional gaussian heatmaps, from which coordinates of keypoint elements within the coordinate system of the field of view of the photosensor imaging system and confidence values may be estimated. But also, other network architectures for the neural network may be suitable.

The keypoint detection module may be made robust against random obstructions, since a typical patient table may be covered with mattresses, paddings, blankets, coil interfaces and other material. For example, the synthetic training data used to train the keypoint detection module may be supplemented with such random obstructions. The resulting robustness may enable a regular checking of the validity of an existing calibration during normal clinical usage.

Using the keypoint detection module, unique points in two dimensions may be identified in the photosensor imaging data acquired using the photosensor imaging system. Additionally, a depth sensing method may be used for determining third coordinates, i.e., distances of the detected keypoint elements from the sensor plane of the photosensor imaging system.

For determining distances of the first keypoint elements of the patient table and/or of the keypoint elements of the imaging phantom the photosensor imaging system may, e.g., comprise a photosensor configured as a depth sensor, or the photosensor imaging system may, e.g., comprise a plurality of imaging photosensors arranged spaced apart from each other and configured determining distances using a stereoscopic method. For example, distances of the first keypoint elements of the patient table and/or of the keypoint elements of the imaging phantom may be determined using triangulation of the first keypoint elements of the patient table and/or of the keypoint elements of the imaging phantom, respectively.

Alternatively, the keypoint detection module may, e.g., be configured to directly determine the third vertical coordinate values of the detected keypoint elements using the photosensor imaging data.

For example, a plurality of photosensor imaging data may be acquired using the photosensor imaging system. The keypoint elements may be detected within the plurality of photosensor imaging data using the keypoint detection module. The photosensor imaging data used for the calibration may, e.g., be selected from the plurality of photosensor imaging data. The selecting may, e.g., comprise a checking that confidence values determined for a predefined number of the keypoint elements detected within the photosensor imaging data being selected exceed a predefined threshold.

Example may have the beneficial effect, that the photosensor imaging data used for the calibration is selected from a plurality of photosensor imaging data received. When selecting the respective photosensor imaging data, it is ensured that the confidence values determined for a predefined number of the keypoint elements detected within the selected photosensor imaging data exceed a predefined threshold. Thus, it may be ensured that the photosensor imaging data used for the calibration is of sufficient quality, in order to ensure a sufficient quality of the resulting calibration. For example, it may be required that the confidence values determined for all the keypoint elements detected within the selected photosensor imaging data exceed the predefined threshold.

For example, the method further comprises determining the first position of the patient table using the determined first keypoint elements. The determined first position of the patient table is used for the determining of the second relative position. For example, a relative position of the second position of the patient table relative to the first position of the patient table is determined and used for determining the second relative position.

For example, the keypoint detection module is further configured for detecting as output one or more keypoint elements of the patient table within the second photosensor imaging data in response to receiving the second photosensor imaging data as input. The method further comprises determining second keypoint elements of the patient table within the second photosensor imaging data using the keypoint detection module. The second position of the patient table is determined using the determined second keypoint elements. The determined second position of the patient table is used for the determining of the second relative position.

For determining distances of the second keypoint elements of the patient table, the photosensor imaging system may, e.g., comprise a photosensor configured as a depth sensor, or the photosensor imaging system may, e.g., comprise a plurality of imaging photosensors arranged spaced apart from each other and configured determining distances using a stereoscopic method. For example, distances of the second keypoint elements of the patient table may be determined using triangulation of the second keypoint elements of the patient table.

For example, the keypoint elements of the patient table are distributed over a surface of patient table, more particularly along a contour line of the patient table.

For example, the keypoint detection module is configured for detecting a number of predefined keypoint elements of the patient table within photosensor imaging data of the patient table. For example, the keypoint elements are elements arranged at an upper side of the patient table. For example, the keypoint elements are elements distributed circumferentially around an upper side of the patient table, e.g., a reclining surface, along a contour line of the upper side of the patient table. The keypoint detection module may, e.g., comprise a neural network trained to detect the pre-defined relevant keypoint elements of the patient table within photosensor imaging data of the patient table.

For example, the keypoint elements of the patient table are distributed over a surface of the patient table. For example, the keypoint elements may be elements distributed across the surface on grid points of an imaginary grid laid over the respective surface.

The surface may, e.g., be an upper surface of the patient table. The photosensor imaging system may, e.g., be arranged above the patient table. The photosensor imaging system may, e.g., be orientated to look vertically downwards onto the upper surface of the patient table. The upper surface may, e.g., be provided with and/or comprise a reclining surface.

For example, the keypoint elements of the patient table are distributed along a contour line of the patient table. The contour line may, e.g., be a contour line of an upper surface of the patient table. Using keypoint elements of the patient table are distributed along a contour line of the patient table may have the beneficial effect, that a likelihood of the keypoint elements being covered, e.g., by a mattress, padding, coil interface or other material arranged on the patient table, may be comparably low. Also, a likelihood of the keypoint elements being covered by a patient lying on the upper surface, e.g., a reclining surface, of the patient table may be comparably low in this case.

For example, the keypoint elements of the imaging phantom are located at a center of the imaging phantom and/or the keypoint elements being distributed over a surface of the imaging phantom.

For example, the method further comprises providing first training datasets for training the keypoint elements detection module. The first training datasets comprise first photosensor imaging training data of the patient table without keypoint elements of the patient table labeled within the first photosensor imaging training data and second photosensor imaging training data of the patient table with the keypoint elements of the patient table labeled within the second photosensor imaging training data. The keypoint detection module is trained for detecting as output the labeled keypoint elements of the patient table of the second photosensor imaging training data of the first training datasets in response to receiving the first photosensor imaging training data of the respective first training datasets. At least a part of the first training datasets comprise second photosensor imaging training data of the imaging phantom with keypoint elements of the imaging phantom labeled additionally within the respective photosensor imaging training data. The method further comprises training the keypoint detection module for further detecting as output the labeled keypoint elements of the imaging phantom of the second photosensor imaging training data of the at least part of the first training datasets in response to receiving the first photosensor imaging training data of the respective first training datasets.

For example, the labels comprise two-dimensional horizontal coordinate values of the respective keypoint elements of the patient table. The two-dimensional horizontal coordinate values may be descriptive of positions of the respective keypoint elements in planes parallel to a sensor plane of the photosensor imaging system. The training may further comprise training the keypoint detection module for determining as output the two-dimensional horizontal coordinate values of the labeled keypoint elements of the patient table within the photosensor imaging training data of the training datasets in response to receiving the photosensor imaging training data of the respective training dataset.

Examples may have the beneficial effect that the keypoint detection module may in addition be trained to determine two-dimensional horizontal coordinate values of the detected keypoint elements.

For example, the labels further comprise third vertical coordinate values of the respective keypoint elements. The third vertical coordinate values are descriptive of distances of the positions of the respective keypoint elements from the sensor plane of the photosensor imaging system. The training may further comprise training the keypoint detection module for determining as output the third vertical coordinate values of the labeled keypoint elements of the patient table within the photosensor imaging training data of the training datasets in response to receiving the photosensor imaging training data of the respective training dataset.

Examples may have the beneficial effect that the keypoint detection module may in addition be trained to determine third vertical coordinate values of the detected keypoint elements.

For example, the keypoint detection module may be trained for determining confidence levels. These confidence levels may, e.g., be estimations trained using a loss function quantifying a deviation of the detections of the keypoint elements from a ground truth for the keypoint elements provided by the photosensor imaging training data. The confidence levels may, e.g., be functions of an output of the keypoint detection module. A maximum, minimum, mean, or integral of the output of the detection module may be examples of quantities, which may be used to derive a confidence metric within a training distribution provided by a plurality of photosensor imaging training data.

For example, the photosensor imaging training data of the imaging phantom, i.e., first photosensor imaging training data of the imaging phantom without keypoint elements of the imaging phantom labeled and second photosensor imaging training data of the imaging phantom with the keypoint elements of the imaging phantom labeled, comprise variations of optical properties of the imaging phantom due to an optical degrading and/or changing of appearance of the imaging phantom with time. The variations of properties of the imaging phantom may, e.g., be generated using simulations.

The keypoint detection module may, e.g., be trained to detect keypoint elements of the patient table using photosensor imaging data acquired of the patient table. The keypoint detection module may, e.g., be trained to detect keypoint elements of the patient table using photosensor imaging data acquired of a three-dimensional physical model of the patient table. The keypoint detection module may, e.g., be trained to detect keypoint elements of the patient table using a three-dimensional digital model of the patient table, e.g., a CAD model of the patient table.

For example, a three-dimensional digital model, e.g., a CAD model, of the patient table is used for generating the training datasets for training the keypoint detection module. Using a three-dimensional digital model may have the beneficial effect that the model may be provided with labels identifying the keypoint elements of the patient table to be detected. Different training datasets may be generated by scaling the three-dimensional digital model and/or generating, e.g., randomly, a background for the three-dimensional digital model. Such a background may, e.g., be generated by a random combination of image data and/or a random processing of image data, like altering colors, altering a color distribution, rotating, translating, adding obstruction to and/or distorting the image data. Also, a position of the three-dimensional digital model may, e.g., be varied.

The keypoint detection module may, e.g., be trained to detect keypoint elements of the imaging phantom using photosensor imaging data acquired of the imaging phantom. The keypoint detection module may, e.g., be trained to detect keypoint elements of the imaging phantom using photosensor imaging data acquired of a three-dimensional physical model of the imaging phantom. The keypoint detection module may, e.g., be trained to detect keypoint elements of the imaging phantom using a three-dimensional digital model of the imaging phantom, e.g., a CAD model of the imaging phantom.

For example, a three-dimensional digital model, e.g., a CAD model, of the imaging phantom is used for generating the second training datasets for training the keypoint detection module. Using a three-dimensional digital model may have the beneficial effect that the model may be provided with labels identifying the keypoint elements of the imaging phantom to be detected. Different training datasets may be generated by scaling the three-dimensional digital model and/or generating, e.g., randomly, a background for the three-dimensional digital model. For example, the three-dimensional digital model of the imaging phantom may be arranged on a three-dimensional digital model of the patient table. A background may, e.g., be generated by a random combination of image data and/or a random processing of image data, like altering colors, altering a color distribution, rotating, translating, adding obstruction to and/or distorting the image data. Also, a position of the three-dimensional digital model of the imaging phantom on the three-dimensional digital model of the patient table may, e.g., be varied. Furthermore, a position of the three-dimensional digital model of the patient table with the three-dimensional digital model of the imaging phantom arranged thereon may, e.g., be varied.

For example, a simulation pipeline is used to generate synthetic training data for training the keypoint detection module.

For example, the computational system further comprises a phantom position detection module configured for determining as output a relative position of the imaging phantom relative to the imaging reference position of the medical imaging system in response to receiving the medical imaging data of the imaging phantom as input. The phantom position detection module is used for the determining of the third relative position of the imaging phantom.

Examples may enable an automatic detection of a position of the imaging phantom using the medical imaging data. For example, a position of a phantom reference point of the imaging phantom in medical image reconstructed using the medical imaging data may be detected. Such medical imaging data may, e.g., be acquired using dedicated and fixed protocols, e.g., CT or MRI protocols. From the acquired medical imaging data medical images may, e.g., be reconstructed and a detection algorithm for detecting the imaging phantom may, e.g., use image processing approaches, like segmentation and/or feature extraction.

Alternatively, training data may be generated, e.g., using a known geometry of the imaging phantom, a known material composition of the imaging phantom and/or a known imaging protocol to be used for acquiring medical imaging data of the imaging phantom. Such training data may, e.g., be used for training a dedicated detection machine learning module, like the phantom position detection module. For example, the dedicated detection module comprises a neural network.

For example, the method further comprises providing second training datasets for training the phantom position detection module. The second training datasets comprise first medical imaging training data of the imaging phantom arranged within the imaging zone of the medical imaging system and second training data identifying relative positions of the imaging phantom arranged within the imaging zone relative to the imaging reference position of the medical imaging system. The phantom position detection module is trained for determining as output the relative positions of the imaging phantom of the second training data of the second training datasets in response to receiving the first medical imaging training data of the respective second training datasets.

For example, the calibrating further comprises a determining of one or more transformation parameters for a transformation between a first coordinate system and a second coordinate system using the determined relative position of the imaging reference position relative to the table reference position. The first coordinate system is a table coordinate system defined relative to the table reference position. The second coordinate system is an imaging coordinate system defined relative to the imaging reference position.

For example, the transformation described by the one or more transformation parameters comprises one or more of the following: one or more rotations, one or more translations, one or more distortions, a scaling.

Since the coordinate systems are three-dimensional, there may be up to three rotations around three perpendicular axes, e.g., extending into the three dimensions, and thus up to three corresponding rotation parameters. There may be up to three translations along three perpendicular axes, e.g., extending into the three dimensions, and thus up to three corresponding translation parameters. The number of distortions as well as the number of corresponding distortion parameters may, e.g., be independent of the number of dimensions. There may, e.g., be one scaling and a corresponding scaling parameter. In case a scaling may vary locally, this variation may, e.g., be described by the distortions.

Examples may, e.g., enable an automatic calibration of a transformation from the table reference position, e.g., a home position of the patient table, to the imaging reference position, e.g., an imaging iso-center of the medical imaging system, for the photosensor imaging system. The photosensor imaging system may, e.g., provide a sensor system for a supervision of an imaging workflow executed using the medical imaging system.

Based on the calibration, it can be determined, which coordinate values within the table coordinate system correspond to which coordinate values within the imaging coordinate system. Using the transformation defined by the one or more determined transformation parameters, it can be determined where a target structure, like a patient and/or a component of the medical imaging system, is positioned with respect to the medical imaging system, i.e., the imaging reference position, based on a position of the target structure with respect to the table and/or with respect to the table reference position, which is determined using photosensor imaging data acquired with the photosensor imaging system. For detecting target structures, i.e., keypoint elements of target structures, a target structure keypoint detection module may be used, which is configured for detecting as output keypoint elements of the target structures within photosensor imaging data in response to receiving the photosensor imaging data acquired using the photosensor imaging system. This target structure keypoint detection module configured for detecting keypoint elements of the target structures may, e.g., be provided in form of the keypoint detection module configured for keypoint elements of the patient table or in form of another keypoint detection module.

For example, the method further comprises controlling a movement of the patient table to a target position defined within the second coordinate system. The controlling comprises receiving third photosensor imaging data acquired using the photosensor imaging system. The third photosensor imaging data comprises photosensor imaging data of the patient table in an initial third position. The third position of the patient table is determined within the first coordinate system using the third photosensor imaging data. The movement required for moving the patient table from the determined third position to the target position is determined using the one or more transformation parameters for the transformation between the first coordinate system and the second coordinate system. An execution of the determined movement is controlled.

To achieve a high precision in determining table positions and controlling table movements relative to the medical imaging system, i.e., the imaging reference position, the table coordinate system needs to be calibrated and registered with respect to a coordinate system of the imaging modality, i.e., the imaging coordinate system.

Such a calibration may be a pre-condition for being able to identify positions of the table and elements arranged thereon relative to the imaging reference position.

Installing a medical imaging system, like an MRI or CT system, may be a time-consuming process by itself and adding any complexity to this process may only increase the challenge. Examples may provide a calibration approach for facilitating this process. Examples may enable a simple and efficient calibration, which may, e.g., be executed together with other installation steps of the medical imaging system, which may, e.g., be executed remotely, and/or which may, e.g., be executed semi of fully automatically.

For example, the computational system further comprises an additional patient table bending model, which is descriptive of a bending the patient table. The bending of the patient table may, e.g., be described as a function of a weight and position of a load arranged on the patient table. For example, the bending model is used for taking into account a bending of the patient table for the controlling of the movement of the patient table. For example, the bending model is used for taking into account a bending of the patient table, when defining the target position within the second coordinate system.

For taking into account a weight induced bending of the patient table, e.g., a table bending model may be used, which is, e.g., a function of weight and position of a center of mass of a weight arranged on the patient table. Such a bending model may, e.g., be obtained from a simulation including relevant forces and mechanical material properties of the patient table. For example, an elastic modulus of a travelling tabletop may be assumed to be finite and homogenous, while a table base may be assumed to be non-deforming, i.e., be described by an infinite modulus. The mass may, e.g., be modelled up to 1^{st} momentum, i.e., as a mass point located at its center of mass. For higher accuracy, e.g., higher orders and/or inhomogeneous material compositions may be used. During calibration, e.g., a known mass and position of the imaging phantoms may be used and fed into the bending model. During an actual imaging and normal patient use, a mass of a patient to be scanned and its center may, e.g., be determined using the photosensor imaging system. Alternatively, e.g., patient information from a radiological information system (RIS) and a default, e.g., centered, patient position may be used. A radiological information system is the core system for the electronic management of medical imaging departments.

For example, a computer program for calibrating a medical imaging system is provided. The calibrating comprises a determining of a relative position of an imaging reference position of the medical imaging system relative to a table reference position of a patient table of the medical imaging system using a photosensor imaging system. The photosensor imaging system is, e.g., configured for a supervision of the medical imaging system.

The computer program comprises machine executable instructions for an execution by a computational system. The execution of the machine executable instructions causes the computational system to perform any of the examples of a method for calibrating the medical imaging system disclosed herein.

For example, a medical system comprising a computational system for calibrating a medical imaging system is provided. The calibrating comprises a determining of a relative position of an imaging reference position of the medical imaging system relative to a table reference position of a patient table of the medical imaging system using a photosensor imaging system. The photosensor imaging system is, e.g., configured for a supervision of the medical imaging system.

The computational system comprises a memory storing machine executable instructions. An execution of the machine executable instructions causes the computational system to perform any of the examples of a method for calibrating the medical imaging system disclosed herein.

For example, the medical system comprises the photosensor imaging system. The photosensor imaging system is, e.g., configured for the supervision of the medical imaging system with the patient table. The machine executable instructions are further configured for controlling the photosensor imaging system to acquire the first and second photosensor imaging data.

The photosensor imaging system may, e.g., comprise one or more imaging photosensors. The photosensor imaging system may be arranged relative to the medical imaging system with the patient table, such that the patient table is contained in the field of view of the photosensor imaging system. For example, the photosensor imaging system may be arranged above the patient table looking from above onto the patient table. For example, the photosensor imaging system may be arranged above a home position of the patient table.

The photosensor imaging system, e.g., in combination with artificial intelligence (AI) algorithms, such as one or more machine learning modules, may be used for guiding and automating patient setups in the medical imaging system. Furthermore, the photosensor imaging systems may, e.g., be used for detecting non-ideal and/or unsafe system uses as well as non-ideal and/or unsafe setup configurations. For example, a position of a patient on the patient table and/or positions of components of the medical imaging system, like cables and/or coils, in particular relative to the patient and/or patient table, may be checked. Furthermore, a positioning of the patient table within the medical imaging system may be controlled based on the detected position of patient, in order to arrange the patient at a predefined position within the medical imaging system for an acquisition of medical imaging data.

When using such AI algorithms, the underlying AI algorithms rely on detecting a patient's body and anatomy, as well as relevant equipment parts of the medical imaging system based on photosensor imaging data acquired using the photosensor imaging system.

In case of a plurality of imaging photosensors with differing fields of view being comprised by the photosensor imaging system, the imaging photosensors may be arranged relative to the medical imaging system with the patient table, such that the patient table is contained in the fields of view of the imaging photosensors of the plurality of imaging photosensors.

For example, the medical system comprises the patient table. The patient table may, e.g., be configured to be moved, e.g., in two or three dimensions, relative to the rest of the medical imaging system, i.e., the imaging reference position.

For example, the medical system comprises the medical imaging system. The machine executable instructions are further configured for controlling the medical imaging system to acquire the medical imaging data of the imaging phantom arranged within the imaging zone of the medical imaging system. For example, the medical imaging system is one of the following: a magnetic resonance imaging system, a computed tomography system, an X-ray imaging system.

The technology described herein may be applied to the use of imaging photosensors in various medical applications, i.e., for various medical imaging systems. The medical imaging system, like e.g., an MRI system, may be comprised by a radiation therapy (RT) system. The X-ray imaging system may, e.g., be part of an image guided therapy (IGT) system. The X-ray imaging system may, e.g., be a diagnostic X-ray (DXR) system.

For example, the medical system comprises the imaging phantom.

An imaging phantom is a specially designed object that may be used in the field of medical imaging to evaluate, analyze, and tune the performance of a medical imaging system. For this purpose, medical imaging data of the imaging phantom may be acquired and analyzed. For example, medical images of the imaging phantom may be reconstructed using the acquired medical imaging data of the imaging phantom and analyzed. The imaging phantom may, e.g., be designed to resemble human tissue of which medical imaging data is to be acquired. Resembling human tissue refers to the fact that medical imaging data acquired of the imaging phantom may resemble medical imaging data acquired of human tissue. Imaging phantoms may, e.g., be created using digital files that are rendered, e.g., through medical imaging and/or computer-aided design (CAD).

For example, the machine executable instructions are further configured for controlling a movement of the patient table.

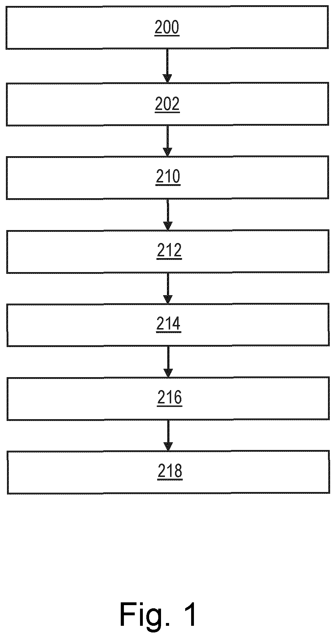

Fig. 1 shows an exemplary method for calibrating a medical imaging system. The calibrating comprises by a computational system a determining of a relative position of an imaging reference position of the medical imaging system relative to a table reference position of a movable patient table using a photosensor imaging system. The photosensor imaging system is, e.g., configured for a supervision of the medical imaging system with the patient table.

In block 200, first photosensor imaging data acquired using the photosensor imaging system is received. The first photosensor imaging data comprises photosensor imaging data of the patient table in an initial first position with an imaging phantom arranged on the patient table. In block 202, a first relative position of the imaging phantom relative to the patient table is determined using the first photosensor imaging data. For example, the computational system comprises a keypoint detection module configured for detecting as output one or more keypoint elements of the patient table within the first photosensor imaging data in response to receiving the first photosensor imaging data as input. The keypoint detection module is, e.g., further configured for detecting as output one or more keypoint elements of the imaging phantom within the first photosensor imaging data received as input.

The method, e.g., further comprises determining first keypoint elements of the patient table within the first photosensor imaging data using the keypoint detection module. Furthermore, keypoint elements of the imaging phantom within the first photosensor imaging data are determined using the keypoint detection module. The determined first keypoint elements of the patient table and the determined keypoint elements of the imaging phantom, e.g., are used for the determining of the first relative position.

For example, the first position of the patient table is determined using the determined first keypoint elements.

For determining distances of the first keypoint elements of the patient table and/or of the keypoint elements of the imaging phantom the photosensor imaging system may, e.g., comprise a photosensor configured as a depth sensor, or the photosensor imaging system may, e.g., comprise a plurality of imaging photosensors arranged spaced apart from each other and configured determining distances using a stereoscopic method. For example, distances of the first keypoint elements of the patient table and/or of the keypoint elements of the imaging phantom may be determined using triangulation of the first keypoint elements of the patient table and/or of the keypoint elements of the imaging phantom, respectively.

In block 210, a second relative position of a second position of the patient table relative to the table reference position is determined. In the second position of the patient table the imaging phantom is arranged within an imaging zone of the medical imaging system.

For example, the first position of the patient table using the first keypoint elements determined with the keypoint detection module. The determined first position of the patient table is used for the determining of the second relative position.

For example, the keypoint detection module is further configured for detecting as output one or more keypoint elements of the patient table within the second photosensor imaging data in response to receiving the second photosensor imaging data as input. Second keypoint elements of the patient table within the second photosensor imaging data are determined using the keypoint detection module. The second position of the patient table is determined using the determined second keypoint elements. The determined second position of the patient table is used for the determining of the second relative position.

For determining distances of the second keypoint elements of the patient table the photosensor imaging system may, e.g., comprise a photosensor configured as a depth sensor, or the photosensor imaging system may, e.g., comprise a plurality of imaging photosensors arranged spaced apart from each other and configured determining distances using a stereoscopic method. For example, distances of the second keypoint elements of the patient table may be determined using triangulation of the second keypoint elements of the patient table.

In block 212, medical imaging data of the imaging phantom arranged within the imaging zone, which is acquired by the medical imaging system, is received. In block 214, a third relative position of the imaging phantom relative to the imaging reference position of the medical imaging system is determined using the medical imaging data. In block 216, the relative position of the imaging reference position of the medical imaging system relative to the table reference position of the patient table is determined using the determined first, second, and third relative position.

For example, the calibrating further comprises a determining of one or more transformation parameters for a transformation between a first coordinate system and a second coordinate system. The first coordinate system is a table coordinate system defined relative to the table reference position. The second coordinate system is an imaging coordinate system defined relative to the imaging reference position. In block 218, the one or more transformation parameters for the transformation between the first coordinate system and the second coordinate system is determined using the determined relative position of the imaging reference position relative to the table reference position.

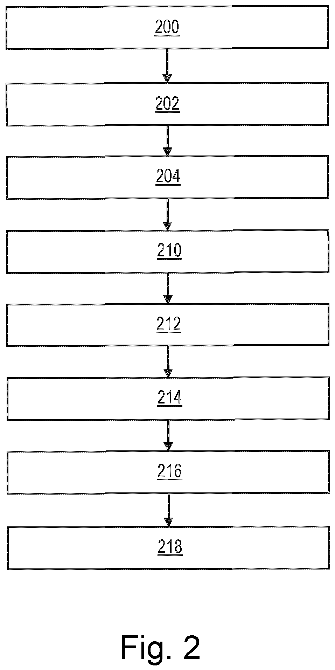

Fig. 2 shows an exemplary method for calibrating a medical imaging system. The calibrating comprises by a computational system a determining of a relative position of an imaging reference position of the medical imaging system relative to a table reference position of a movable patient table using a photosensor imaging system. The photosensor imaging system is, e.g., configured for a supervision of the medical imaging system with the patient table.

In block 200, first photosensor imaging data acquired using the photosensor imaging system is received. The first photosensor imaging data comprises photosensor imaging data of the patient table in an initial first position with an imaging phantom arranged on the patient table. In block 202, a first relative position of the imaging phantom relative to the patient table is determined using the first photosensor imaging data. For example, the computational system comprises a keypoint detection module configured for detecting as output one or more keypoint elements of the patient table within the first photosensor imaging data in response to receiving the first photosensor imaging data as input. The keypoint detection module is, e.g., further configured for detecting as output one or more keypoint elements of the imaging phantom within the first photosensor imaging data received as input.

The method, e.g., further comprises determining first keypoint elements of the patient table within the first photosensor imaging data using the keypoint detection module. Furthermore, keypoint elements of the imaging phantom within the first photosensor imaging data are determined using the keypoint detection module. The determined first keypoint elements of the patient table and the determined keypoint elements of the imaging phantom, e.g., are used for the determining of the first relative position.

For example, the first position of the patient table is determined using the determined first keypoint elements.

For determining distances of the first keypoint elements of the patient table and/or of the keypoint elements of the imaging phantom the photosensor imaging system may, e.g., comprise a photosensor configured as a depth sensor, or the photosensor imaging system may, e.g., comprise a plurality of imaging photosensors arranged spaced apart from each other and configured determining distances using a stereoscopic method. For example, distances of the first keypoint elements of the patient table and/or of the keypoint elements of the imaging phantom may be determined using triangulation of the first keypoint elements of the patient table and/or of the keypoint elements of the imaging phantom, respectively.

In block 204, second photosensor imaging data acquired using the photosensor imaging system is received. The second photosensor imaging data comprises photosensor imaging data of the patient table moved to a second position with the imaging phantom arranged within an imaging zone of the medical imaging system. In block 210, a second relative position of the second position of the patient table relative to the table reference position is determined using the second photosensor imaging data.

For example, the first position of the patient table using the first keypoint elements determined with the keypoint detection module. The determined first position of the patient table is used for the determining of the second relative position.

For example, the keypoint detection module is further configured for detecting as output one or more keypoint elements of the patient table within the second photosensor imaging data in response to receiving the second photosensor imaging data as input. Second keypoint elements of the patient table within the second photosensor imaging data are determined using the keypoint detection module. The second position of the patient table is determined using the determined second keypoint elements. The determined second position of the patient table is used for the determining of the second relative position.

For determining distances of the second keypoint elements of the patient table the photosensor imaging system may, e.g., comprise a photosensor configured as a depth sensor, or the photosensor imaging system may, e.g., comprise a plurality of imaging photosensors arranged spaced apart from each other and configured determining distances using a stereoscopic method. For example, distances of the second keypoint elements of the patient table may be determined using triangulation of the second keypoint elements of the patient table.

In block 212, medical imaging data of the imaging phantom arranged within the imaging zone, which is acquired by the medical imaging system, is received. In block 214, a third relative position of the imaging phantom relative to the imaging reference position of the medical imaging system is determined using the medical imaging data. In block 216, the relative position of the imaging reference position of the medical imaging system relative to the table reference position of the patient table is determined using the determined first, second, and third relative position.

For example, the calibrating further comprises a determining of one or more transformation parameters for a transformation between a first coordinate system and a second coordinate system. The first coordinate system is a table coordinate system defined relative to the table reference position. The second coordinate system is an imaging coordinate system defined relative to the imaging reference position. In block 218, the one or more transformation parameters for the transformation between the first coordinate system and the second coordinate system is determined using the determined relative position of the imaging reference position relative to the table reference position.

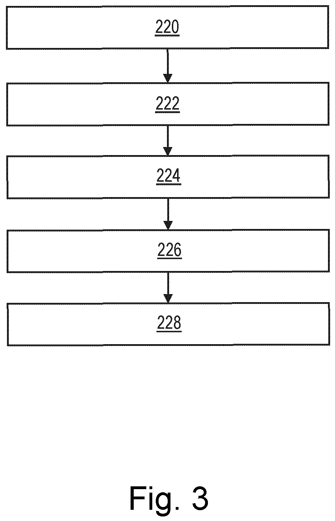

Fig. 3 shows an exemplary method for controlling a movement of the movable patient table using the relative position of the imaging reference position relative to the table reference position determined in Fig. 2. The patient table is controlled to be moved to a target position defined within the second coordinate system, i.e., the imaging coordinate system defined relative to the imaging reference position. In block 220, the target position is defined within the second coordinate system.

For example, the computational system further comprises an additional patient table bending model, which is descriptive of a bending the patient table. The bending of the patient table may, e.g., be described as a function of a weight and position of a load arranged on the patient table. For example, the bending model is used for taking into account a bending of the patient table for the controlling of the movement of the patient table. For example, the bending model is used for taking into account a bending of the patient table, when defining the target position within the second coordinate system.

In block 222, third photosensor imaging data acquired using the photosensor imaging system is received. The third photosensor imaging data comprises photosensor imaging data of the patient table in an initial third position. In block 224, the third position of the patient table is determined within the first coordinate system using the third photosensor imaging data. In block 226, the movement required for moving the patient table from the determined third position to the target position is determined using the one or more transformation parameters for the transformation between the first coordinate system and the second coordinate system. In block 228, an execution of the determined movement is controlled.

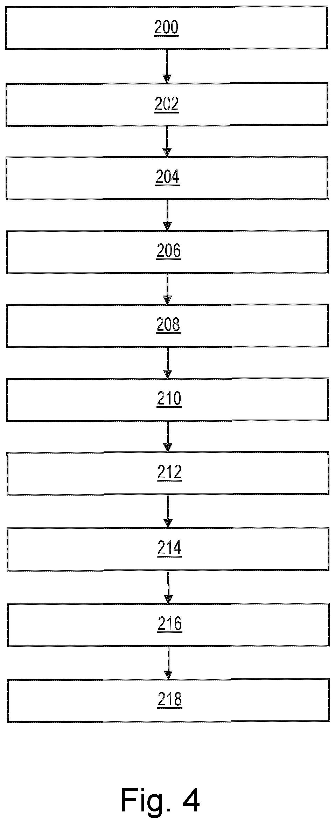

Fig. 4 shows an exemplary method for calibrating a medical imaging system. The calibrating comprises by a computational system a determining of a relative position of an imaging reference position of the medical imaging system relative to a table reference position of a movable patient table using a photosensor imaging system. The photosensor imaging system is, e.g., configured for a supervision of the medical imaging system with the patient table.

In block 200, first photosensor imaging data acquired using the photosensor imaging system is received. The first photosensor imaging data comprises photosensor imaging data of the patient table in an initial first position with an imaging phantom arranged on the patient table. In block 202, a first relative position of the imaging phantom relative to the patient table is determined using the first photosensor imaging data. For example, the computational system comprises a keypoint detection module configured for detecting as output one or more keypoint elements of the patient table within the first photosensor imaging data in response to receiving the first photosensor imaging data as input. The keypoint detection module is, e.g., further configured for detecting as output one or more keypoint elements of the imaging phantom within the first photosensor imaging data received as input.

The method, e.g., further comprises determining first keypoint elements of the patient table within the first photosensor imaging data using the keypoint detection module. Furthermore, keypoint elements of the imaging phantom within the first photosensor imaging data are determined using the keypoint detection module. The determined first keypoint elements of the patient table and the determined keypoint elements of the imaging phantom, e.g., are used for the determining of the first relative position.

For example, the first position of the patient table is determined using the determined first keypoint elements.

For determining distances of the first keypoint elements of the patient table and/or of the keypoint elements of the imaging phantom the photosensor imaging system may, e.g., comprise a photosensor configured as a depth sensor, or the photosensor imaging system may, e.g., comprise a plurality of imaging photosensors arranged spaced apart from each other and configured determining distances using a stereoscopic method. For example, distances of the first keypoint elements of the patient table and/or of the keypoint elements of the imaging phantom may be determined using triangulation of the first keypoint elements of the patient table and/or of the keypoint elements of the imaging phantom, respectively.

In block 204, second photosensor imaging data acquired using the photosensor imaging system is received. The second photosensor imaging data comprises photosensor imaging data of the patient table moved to a second position with the imaging phantom arranged within an imaging zone of the medical imaging system.

For example, the initial first position differs from the table reference position. In block 206, a fourth relative position of the first position of the patient table relative to the table reference position is determined. In block 208, a fifth relative position of the second position of the patient table relative to the first position of the patient table using the second photosensor imaging data is determined. In block 210, a second relative position of the second position of the patient table relative to the table reference position is determined using the fourth and fifth relative position.

For example, the first position of the patient table using the first keypoint elements determined with the keypoint detection module. The determined first position of the patient table is used for the determining of the second relative position.

For example, the keypoint detection module is further configured for detecting as output one or more keypoint elements of the patient table within the second photosensor imaging data in response to receiving the second photosensor imaging data as input. Second keypoint elements of the patient table within the second photosensor imaging data are determined using the keypoint detection module. The second position of the patient table is determined using the determined second keypoint elements. The determined second position of the patient table is used for the determining of the second relative position.

For determining distances of the second keypoint elements of the patient table the photosensor imaging system may, e.g., comprise a photosensor configured as a sensor, or the photosensor imaging system may, e.g., comprise a plurality of imaging photosensors arranged spaced apart from each other and configured determining distances using a stereoscopic method. For example, distances of the second keypoint elements of the patient table may be determined using triangulation of the second keypoint elements of the patient table.

In block 212, medical imaging data of the imaging phantom arranged within the imaging zone, which is acquired by the medical imaging system, is received. In block 214, a third relative position of the imaging phantom relative to the imaging reference position of the medical imaging system is determined using the medical imaging data.

For example, the computational system further comprises a phantom position detection module configured for determining as output a relative position of the imaging phantom relative to the imaging reference position of the medical imaging system in response to receiving the medical imaging data of the imaging phantom as input. The phantom position detection module is used for the determining of the third relative position of the imaging phantom.

In block 216, the relative position of the imaging reference position of the medical imaging system relative to the table reference position of the patient table is determined using the determined first, second, and third relative position.

For example, the calibrating further comprises a determining of one or more transformation parameters for a transformation between a first coordinate system and a second coordinate system. The first coordinate system is a table coordinate system defined relative to the table reference position. The second coordinate system is an imaging coordinate system defined relative to the imaging reference position. In block 218, the one or more transformation parameters for the transformation between the first coordinate system and the second coordinate system is determined using the determined relative position of the imaging reference position relative to the table reference position.

For example, the relative position of the imaging reference position relative to the table reference position may be used for controlling a movement of the movable patient table using the method of Fig. 3. The patient table is, e.g., controlled to be moved to a target position defined within the second coordinate system, i.e., the imaging coordinate system defined relative to the imaging reference position.

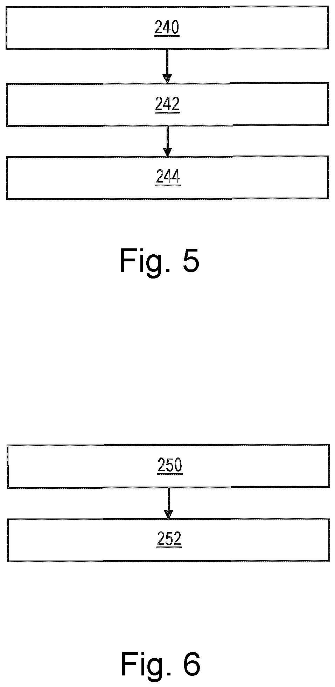

Fig. 5 shows an exemplary method for training a keypoint elements detection module. In block 240, first training datasets are provided for training the keypoint elements detection module. The first training datasets comprise first photosensor imaging training data of the patient table without keypoint elements of the patient table labeled within the first photosensor imaging training data and second photosensor imaging training data of the patient table with the keypoint elements of the patient table labeled within the second photosensor imaging training data. In bock 242, the keypoint detection module is trained for detecting as output the labeled keypoint elements of the patient table of the second photosensor imaging training data of the first training datasets in response to receiving the first photosensor imaging training data of the respective first training datasets.

For example, at least a part of the first training datasets comprise second photosensor imaging training data of the imaging phantom with keypoint elements of the imaging phantom labeled additionally within the respective photosensor imaging training data. In block 244, the keypoint detection module is trained for further detecting as output the labeled keypoint elements of the imaging phantom of the second photosensor imaging training data of the at least part of the first training datasets in response to receiving the first photosensor imaging training data of the respective first training datasets.

For example, the photosensor imaging training data of the imaging phantom, i.e., first photosensor imaging training data of the imaging phantom without keypoint elements of the imaging phantom labeled and second photosensor imaging training data of the imaging phantom with the keypoint elements of the imaging phantom labeled, comprise variations of optical properties of the imaging phantom due to an optical degrading and/or changing of appearance of the imaging phantom with time. The variations of properties of the imaging phantom may, e.g., be generated using simulations.

For example, the labels photosensor imaging training data may comprise two-dimensional horizontal coordinate values of the respective keypoint elements of the patient table. The two-dimensional horizontal coordinate values may be descriptive of positions of the respective keypoint elements in planes parallel to a sensor plane of the photosensor imaging system. The training may further comprise training the keypoint detection module for determining as output the two-dimensional horizontal coordinate values of the labeled keypoint elements of the patient table within the photosensor imaging training data of the training datasets in response to receiving the photosensor imaging training data of the respective training dataset.

For example, the labels may further comprise third vertical coordinate values of the respective keypoint elements. The third vertical coordinate values are descriptive of distances of the positions of the respective keypoint elements from the sensor plane of the photosensor imaging system. The training may further comprise training the keypoint detection module for determining as output the third vertical coordinate values of the labeled keypoint elements of the patient table within the photosensor imaging training data of the training datasets in response to receiving the photosensor imaging training data of the respective training dataset.

For example, the keypoint detection module may be trained for determining confidence levels. These confidence levels may, e.g., be estimations trained using a loss function quantifying a deviation of the detections of the keypoint elements from a ground truth for the keypoint elements provided by the photosensor imaging training data. The confidence levels may, e.g., be functions of an output of the keypoint detection module. A maximum, minimum, mean, or integral of the output of the detection module may be examples of quantities, which may be used to derive a confidence metric within a training distribution provided by a plurality of photosensor imaging training data.

Fig. 6 shows an exemplary method for training a phantom position detection module. In block 250, second training datasets are provided for training the phantom position detection module. The second training datasets comprise first medical imaging training data of the imaging phantom arranged within the imaging zone of the medical imaging system and second training data identifying relative positions of the imaging phantom arranged within the imaging zone relative to the imaging reference position of the medical imaging system. In block 252, the phantom position detection module is trained for determining as output the relative positions of the imaging phantom of the second training data of the second training datasets in response to receiving the first medical imaging training data of the respective second training datasets.

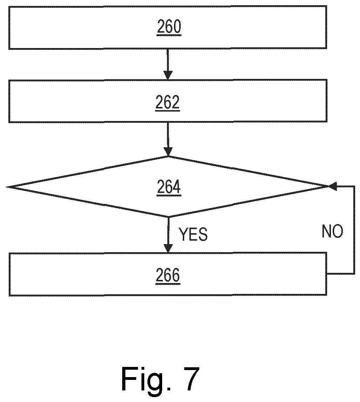

Fig. 7 illustrates a method for selecting photosensor imaging data to be used for a calibration, e.g., a calibration according to any of the methods of Fig. 1 to Fig. 4. In block 260, a plurality of photosensor imaging data acquired using the photosensor imaging system is received. The plurality of photosensor imaging data comprises a plurality of photosensor imaging data of the patient table. In block 262, keypoint elements are detected within the received photosensor imaging data using the keypoint detection module. In block 264, the received plurality of photosensor imaging data is checked, whether confidence values determined for a predefined number of the keypoint elements detected within photosensor imaging data of the plurality of photosensor imaging data exceeds a predefined threshold. In case, it is determined that a predefined number of the keypoint elements detected within photosensor imaging data being checked exceeds the predefined threshold, the respective photosensor imaging data is selected in block 266 to be used for the calibration. In case, it is determined that a predefined number of the keypoint elements detected within photosensor imaging data being checked exceeds the predefined threshold, the method continues in block 264 with checking next photosensor imaging data of the plurality of photosensor imaging data.

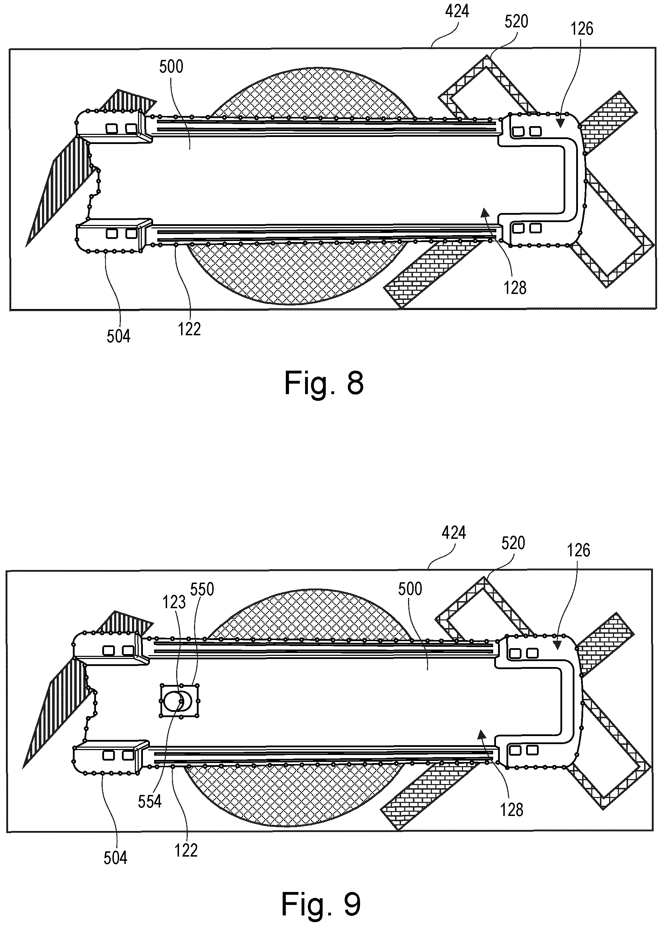

Fig. 8 illustrates exemplary photosensor imaging training data 424 for training a keypoint detection module to detect keypoint elements 122 of a patient table. The photosensor imaging training data 424 is synthetic training data generated using a three-dimensional digital model 500 of the patient table. The three-dimensional digital model 500 comprises an upper surface 126 with a reclining surface 128. The keypoint elements 122 are, e.g., distributed on the upper surface 126. The keypoint elements 122 are, e.g., distributed along a contour line of the patient table circumferentially around the reclining surface 128. The photosensor imaging training data 424 may, e.g., be generated by adding random image elements 520. The random image elements 520 may, e.g., be added in the background of the three-dimensional digital model 500. For example, the random image elements 520 may also be added, at least partially, over the three-dimensional digital model 500 as random obstructions, such that at least some of the keypoint elements 122 are covered by the random image elements 520. Thereby, the keypoint detection module may be made robust against random obstructions, since a typical patient table may be covered additional material, like, e.g., mattresses, paddings, blankets, coil interfaces and other material.

In addition, the photosensor imaging training data 424 comprises labels 504 labeling the keypoint elements 122 within the photosensor imaging training data 424. For a training of the keypoint detection module, e.g., additional photosensor imaging training data 424 may be provided without labels 504 of the keypoint elements 122 as input for the keypoint detection module during training. The keypoint detection module may be trained to detect in the photosensor imaging training data 424 provided without labels 504 the keypoint elements 122, which are identified by the labels 504 of the labeled photosensor imaging training data 424.

For example, the labels 504 may comprise two-dimensional horizontal coordinate values of the respective keypoint elements 122 of the patient table. The two-dimensional horizontal coordinate values may be descriptive of positions of the respective keypoint elements 122 in planes parallel to a sensor plane of the photosensor imaging system. Thus, the photosensor imaging training data 424 may, e.g., also be used for training the keypoint detection module for determining the two-dimensional horizontal coordinate values of the labeled keypoint elements 122 of the patient table as output.

For example, the labels 504 may further comprise third vertical coordinate values of the respective keypoint elements 122. The third vertical coordinate values are descriptive of distances of the positions of the respective keypoint elements 122 from the sensor plane of the photosensor imaging system. Thus, the photosensor imaging training data 424 may, e.g., also be used for training the keypoint detection module for determining the third vertical coordinate values in addition to the two-dimensional horizontal coordinate values of the labeled keypoint elements 122 of the patient table as output.

Fig. 9 illustrates exemplary photosensor imaging training data 44 for training a keypoint detection module to detect keypoint elements 123 of an imaging phantom. The photosensor imaging training data 424 is synthetic training data generated using a three-dimensional digital model 550 of the imaging phantom. Furthermore, the photosensor imaging training data 424 may comprise a three-dimensional digital model 500 of the patient table as shown in Fig. 8, on which the three-dimensional digital model 550 of the imaging phantom is arranged. The keypoint elements 123 may, e.g., comprise a keypoint elements 123 located at a center of the imaging phantom. For example, keypoint elements 123 of the imaging phantom may be distributed over a surface of imaging phantom and/or distributed along a contour line of a holder of the imaging phantom. Like the photosensor imaging training data 424 of Fig. 8, the photosensor imaging training data 424 may, e.g., be generated by adding random image elements 520. The random image elements 520 may, e.g., be added in the background of the three-dimensional digital model 500. For example, the random image elements 520 may also be added, at least partially, over the three-dimensional digital model 500 as random obstructions, such that at least some of the keypoint elements 122 are covered by the random image elements 520. Thereby, the keypoint detection module may be made robust against random obstructions, since a typical patient table may be covered additional material, like, e.g., mattresses, paddings, blankets, coil interfaces and other material.

In addition, the photosensor imaging training data 424 comprises labels 554 labeling the keypoint elements 132 of the imaging phantom within the photosensor imaging training data 424. For a training of the keypoint detection module, e.g., additional photosensor imaging training data 424 may be provided without labels 554 of the keypoint elements 123 as input for the keypoint detection module during training. The keypoint detection module may be trained to detect in the photosensor imaging training data 424 provided without labels 554 the keypoint elements 123, which are identified by the labels 554 of the labeled photosensor imaging training data 424.

For example, the labels 554 may comprise two-dimensional horizontal coordinate values of the respective keypoint elements 123 of the imaging phantom. The two-dimensional horizontal coordinate values may be descriptive of positions of the respective keypoint elements 123 in planes parallel to a sensor plane of the photosensor imaging system. Thus, the photosensor imaging training data 424 may, e.g., also be used for training the keypoint detection module for determining the two-dimensional horizontal coordinate values of the labeled keypoint elements 123 of the imaging phantom as output.

For example, the labels 554 may further comprise third vertical coordinate values of the respective keypoint elements 123. The third vertical coordinate values are descriptive of distances of the positions of the respective keypoint elements 123 from the sensor plane of the photosensor imaging system. Thus, the photosensor imaging training data 424 may, e.g., also be used for training the keypoint detection module for determining the third vertical coordinate values in addition to the two-dimensional horizontal coordinate values of the labeled keypoint elements 123 of the imaging phantom as output.

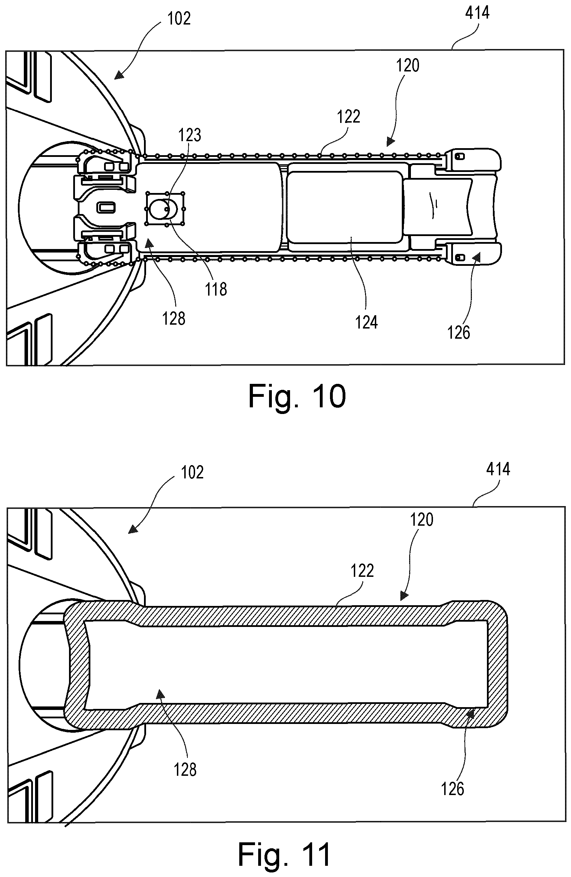

Fig. 10 illustrates an exemplary detection of keypoint elements 122 in photosensor imaging data 414 of a patient table 120. The patient table 120 may, e.g., be part of a medical imaging system, e.g., an MRI system. The patient table 120 comprises an upper surface 126 with a reclining surface 128. The reclining surface is configured for a patient to lie thereon. The keypoint elements 122 of the patient table 120 are, e.g., distributed on the upper surface 126. The keypoint elements 122 of the patient table 120 are, e.g., distributed along a contour line of the patient table circumferentially around the reclining surface 128. Furthermore, additional material 124, like, e.g., mattresses, paddings, blankets, coil interfaces and other material, may be arranged on the patient table 120. The keypoint detection module is configured to detect the keypoint elements 122 in the photosensor imaging data 414.

Fig. 11 illustrates another exemplary detection of keypoint elements 122 in photosensor imaging data 414. The photosensor imaging data 414 of Fig. 11 comprises an empty patient table 120 of a medical imaging system 102 with an upper surface 126 with a reclining surface 128. The detected keypoint elements 122 are represented as gaussian probability heatmaps, e.g., with varying color overlaid over a table image. A size of the individual heatmaps may be such that neighboring keypoint elements have overlapping signal tails. Due to the overlapping, the band-like structure schematically shown in Fig. 11 is formed, which represents the detected keypoint elements 122. The same effect may occur, when detecting keypoint elements of an imaging phantom (not shown).

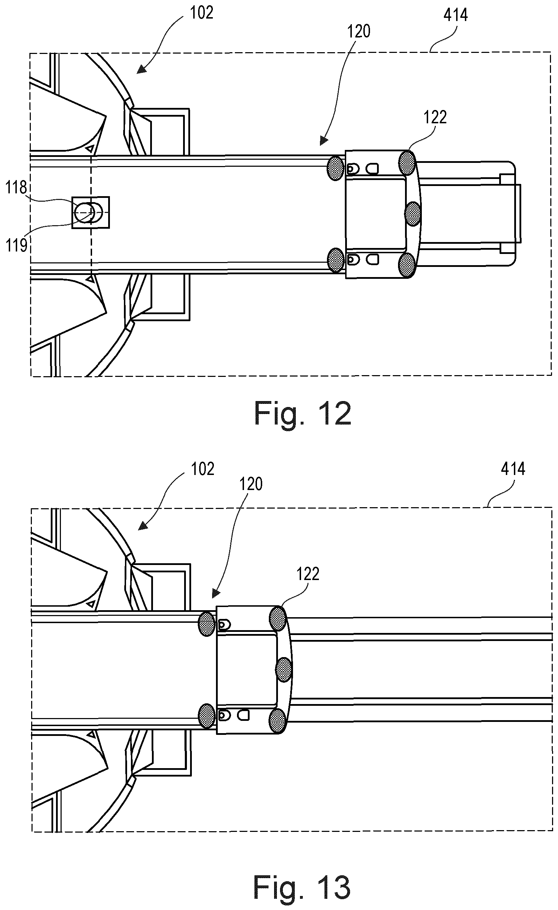

Fig. 12 illustrates exemplary photosensor imaging data 414. The photosensor imaging data 414 of Fig. 12 comprise a patient table 120 with an imaging phantom 118 arranged thereon. The exemplary position of the patient table 120 illustrated in Fig. 12 is a position, in which the imaging phantom 118 is arranged in a position defined by a light visor cross 119 of the medical imaging system 102. The patient table 120 with the imaging phantom 118 may take this position, e.g., on its way from an initial first position of the patient table 120 to a second position of the patient table 120, in which the imaging phantom 118 is arranged within an imaging zone of the medical imaging system 102. An arranging of the patient table 120 in this position may, e.g., be controlled automatically using photosensor imaging data acquired with the photosensor imaging system. The patient table 120 may, e.g., moved from the initial first position to a position, in which the imaging phantom 118 is aligned with the position defined by the light visor cross, i.e. the position illustrated in Fig. 12. For example, the position illustrated in Fig. 12 may be an initial first position of the patient table 120, from which the patient table 120 is moved to the second position of the patient table 120, in which the imaging phantom 118 is arranged within an imaging zone of the medical imaging system 102. The position of the patient table 120 in Fig. 12 is, e.g., determined by detecting keypoint elements 122 of the patient table 120 in the photosensor imaging data 414, e.g., using a keypoint detection module. The detected keypoint elements 122 in Fig. 12 are represented as gaussian probability heatmaps, e.g., with varying color overlaid over a table image. The detected keypoint elements 122 are therefore spatially expanded representing probability distributions determined for these keypoint elements 122. By detecting the keypoint elements 122, the position of the patient table 120 with respect to a table reference position may be determined.

Fig. 13 illustrates exemplary photosensor imaging data 414 comprising the patient table 120 with the imaging phantom 118 (not shown) of Fig. 12 in a position, in the imaging phantom 118 is arranged within an imaging zone of the medical imaging system 102. Since the imaging phantom 118 is arranged within an imaging zone of the medical imaging system 102, e.g., within a gantry of the medical imaging system 102, only part of the patient table 120 may be visible in the photosensor imaging data 414 acquired by the photosensor imaging system. The visible part of the patient table 120 comprises keypoint elements 122, which are detectible within the photosensor imaging data 414. By detecting the keypoint elements 122, the position of the patient table 120 with respect to a table reference position may be determined. The detected keypoint elements 122 in Fig. 13 are represented as gaussian probability heatmaps, e.g., with varying color overlaid over a table image. The detected keypoint elements 122 are therefore spatially expanded representing probability distributions determined for these keypoint elements 122.

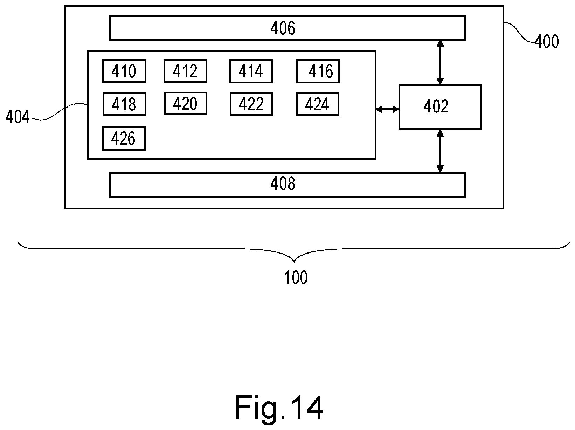

Fig. 14 illustrates an exemplary computational system 400 of a medical system. The computational system 400 is configured for calibrating a medical imaging system. The calibrating comprises a determining of a relative position of an imaging reference position of the medical imaging system relative to a table reference position of a patient table of the medical imaging system using a photosensor imaging system. The photosensor imaging system may, e.g., be configured for a supervision of the medical imaging system. The computational system 400 is shown as comprising a computational component 402. The computational component 402 is intended to represent one or more processors or processing cores or other computational elements. The computational component 402 is shown as being connected to a hardware interface 406 and a memory 404. The hardware interface 406 may enable the computational component 402 to exchange commands and data with other components, e.g., the photosensor imaging system, the medical imaging system, and/or drive device for moving the patient table. The hardware interface 406 may, e.g., enable the computational component 402 to control the photosensor imaging system to acquire the photosensor imaging data. The hardware interface 406 may, e.g., further enable the computational component 402 to control the medical imaging system to acquire medical imaging data. The hardware interface 406 may, e.g., further enable the computational component 402 to control the drive device to move the patient table.

The computational system 404 is further shown as being connected to a user interface 408 which may for example enable an operator to control and operate the computational system 400 and via the computational system 400 the photosensor, the medical imaging system, and/or the drive device. The user interface 408 may, e.g., comprise an output and/or input device enabling an operator to interact with the computational system 400. The output device may, e.g., comprise a display device configured for displaying photosensor imaging data 414 and/or medical imaging data 416. The input device may, e.g., comprise a keyboard and/or a mouse enabling the user to insert control commands for controlling the computational system 400 and via the computational system 400 the photosensor imaging system, the medical imaging system, and/or the driving device.

The memory 404 is shown as containing machine-executable instructions 410. The machine-executable instructions 410 enable the computational component 402 to perform controlling tasks, such as controlling the photosensor imaging system, the medical imaging system, and/or the driving device, to perform numerical tasks, as well as to perform various signal data processing tasks. The machine-executable instructions 410 may, e.g., enable the computational component 402 and thus the computational system 400 to calibrate the medical imaging system according to the methods of Fig. 1 to Fig. 4. The machine-executable instructions 410 may, e.g., enable the computational component 402 and thus the computational system 400 to further execute the methods of Fig. 5 to 7 and/or to generate synthetic photosensor imaging training data 418 according to Fig. 8 and/or Fig. 9.

The memory 404 is further shown as containing a keypoint detection module 412. The keypoint detection module 412 is configured for detecting keypoint elements of one or more target structures within photosensor imaging data 414 in response to receiving the photosensor imaging data 414 from the photosensor. For example, keypoint detection module 412 may be configured for detecting as output one or more keypoint elements of the patient table within the photosensor imaging data 141 in response to receiving the first photosensor imaging data 414 as input. For example, the keypoint detection module 412 may be configured for detecting as output one or more keypoint elements of the imaging phantom within photosensor imaging data 141 received as input.

The keypoint detection module 412 may, e.g., be configured to determine two-dimensional horizontal coordinate values of the detected keypoint elements. The two-dimensional horizontal coordinate values may be descriptive of positions of the detected keypoint elements in planes parallel to a sensor plane of the photosensor imaging system. For example, the keypoint detection module 412 may be configured to determine in addition to the two-dimensional horizontal coordinate values third vertical coordinate values of the detected keypoint elements. The third vertical coordinate values are descriptive of distances of the positions of the detected keypoint elements from the sensor plane of the photosensor imaging system.

The memory 404 is further shown as containing photosensor imaging data 414. The photosensor imaging data 414 is acquired using a photosensor configured for a supervision of a medical imaging system.

The memory 404 is further shown as containing medical imaging data 416 of an imaging phantom arranged within an imaging zone, which is acquired by the medical imaging system.

The memory 404 is further shown as containing a phantom position detection module 418 configured for determining as output a relative position of an imaging phantom relative to the imaging reference position of the medical imaging system in response to receiving the medical imaging data of the imaging phantom as input.

The memory 404 is further shown as containing a specification 420 of a relative position of an imaging reference position of the medical imaging system, e.g., an imaging iso-center, relative to a table reference position of the patient table, e.g., a home position of the patient table.

The memory 404 is further shown as containing one or more transformation parameters 420 for a transformation between a first coordinate system and a second coordinate system. The one or more transformation parameters 422 are determined using the specification 420 of the relative position of the imaging reference position relative to the table reference position. The first coordinate system is a table coordinate system defined relative to the table reference position. The second coordinate system is an imaging coordinate system defined relative to the imaging reference position.

The memory 404 may optionally further contain training datasets 424, e.g., synthetic training data, configured for training the keypoint detection module 412 to detect keypoint elements of one or more target structures, e.g., a patient table and/or an imaging phantom, within the photosensor imaging data 414 in response to receiving the photosensor imaging data 414 from the photosensor.

The memory 404 may optionally further contain training datasets 426, e.g., synthetic training data, configured for training the phantom position detection module 418 to determine a relative position of the imaging phantom arranged within the imaging zone relative to an imaging reference position of the medical imaging system.

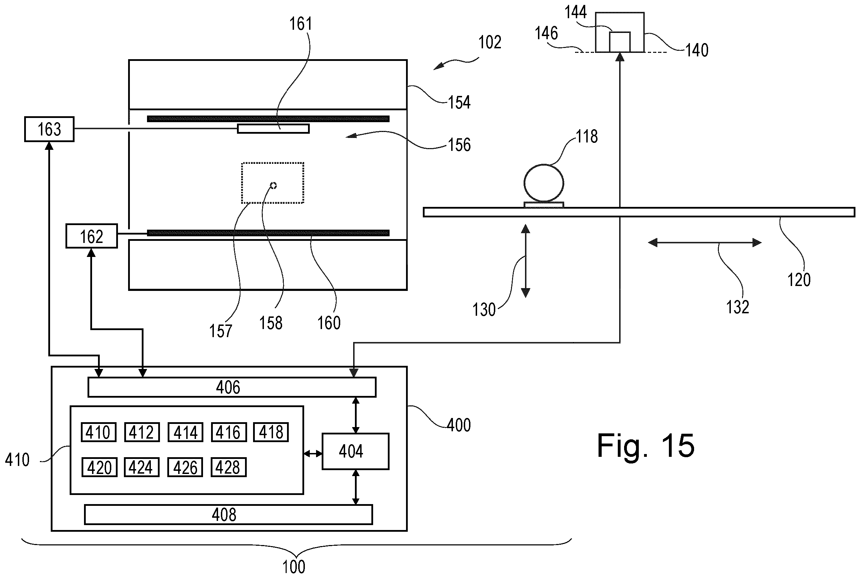

Fig. 15 an exemplary medical system 100 comprising a computational system 400, a photosensor imaging system 140, and a medical imaging system 102. The computational system 400 is configured for calibrating the medical imaging system 102. The computational system 400 of Fig. 15 is, e.g., the computational system 400 of Fig. 14.

The photosensor imaging system 140 may comprise one or more imaging photosensors 144 and may, e.g., be configured for a supervision of the medical imaging system 102 with the patient table 120.

The medical imaging system 102, e.g., comprises a patient table 120. The medical imaging system 102 may, e.g., be a magnetic resonance imaging system. Indicated is also a sensor plane 146 of the photosensor imaging system 140.

Alternatively, the medical imaging system 102 may, e.g., comprise a computed tomography system or an X-ray imaging system. The medical imaging system 102, e.g., with the MRI system, may be comprised by a radiation therapy (RT) system. An X-ray imaging system may, e.g., be part of an image guided therapy (IGT) system or the X-ray imaging system may, e.g., be a diagnostic X-ray (DXR) system.

An imaging phantom 118 is shown as being arranged on the patient table 120. The patient table 120 is movable in at least two spatial directions 130, 132. For example, the patient table 120 is movable in a vertical direction 130. For example, the patient table 120 is movable in a horizontal direction 132. By moving the patient table 120 in the vertical direction 132, the imaging phantom 118 arranged on the patient table 120 is, e.g., moved into a gantry of the medical imaging system 102. For example, the patient table 120 may be movable in three spatial directions.

The medical imaging system 102 depicted in Fig. 15 is implemented in form of a magnetic resonance imaging (MRI) system. The MRI system 102 is controlled by the computer 400. The MRI system 102 comprises a magnet 154. The magnet 154 is a superconducting cylindrical type magnet with a bore 156 through it. The use of different types of magnets is also possible. For instance, it is also possible to use both a split cylindrical magnet and a so-called open magnet.

Within the bore 156 of the cylindrical magnet 154 there is an imaging zone 157, where the magnetic field is strong and uniform enough to perform magnetic resonance imaging. The imaging zone 157 may, e.g., comprise a field of view, for which magnetic resonance data is typically acquired. Within the imaging zone 157 there may, e.g., be an imaging iso-center 158 of the MRI system 102. In the imaging iso-center 158 is a center point within the magnetic field of the MRI system 102. This imaging iso-center 158 represents a central area where the magnetic field is most uniform and where the gradients are most linear.

Within the bore 156 of the magnet there is also a set of magnetic field gradient coils 160 which is used for acquisition of preliminary magnetic resonance data to spatially encode magnetic spins within the bore 156 of magnet 154. The magnetic field gradient coils 160 are connected to a magnetic field gradient coil power supply 162. The magnetic field gradient coils 160 are intended to be representative. Typically, magnetic field gradient coils 160 contain three separate sets of coils for spatially encoding in three orthogonal spatial directions. The magnetic field gradient power supply 162 supplies current to the magnetic field gradient coils 160. The current supplied to the magnetic field gradient coils 160 is controlled as a function of time and may be ramped or pulsed.

Furthermore, the MRI system 102 may comprise an RF transmitter coil 161 for manipulating the orientations of magnetic spins. The RF transmitter coil 161 may also be referred to as a transmitter antenna. The RF transmitter coil 161 is connected to an RF transmitter 163. It is understood that the RF transmitter coil 161 and the RF transmitter 163 are representative. The RF transmitter coil 161 may have multiple transmitter elements and the RF transmitter 163 may have multiple transmitter channels.

The transmitter 163, the gradient controller 162 and the one or more imaging photosensors 144 are shown as being connected to the hardware interface 406 of the computer 400. Furthermore, the hardware interface 406 of the computer 400 may, e.g., be connected to a drive device

(not shown) configured for moving the patient table 120 in one or more spatial directions 130, 132. The computational system 400 is intended to represent one or more computational or computational systems. The computational system 400 is configured for acquiring medical imaging data 416 as part of a control system of the medical imaging system 102, e.g., MRI data using the medical imaging system 102 in form of an MRI system. The MRI data may be used for reconstructing MR images. The computational system 400 is further configured for controlling the one or more imaging photosensors 144 of the photosensor imaging system 140 for acquiring photosensor imaging data 414.

It is understood that one or more of the aforementioned embodiments of the invention may be combined as long as the combined embodiments are not mutually exclusive.

As will be appreciated by one skilled in the art, aspects of the present invention may be embodied as an apparatus, method, or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computational system. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computational system. Examples of computer-readable storage media include, but are not limited to: a floppy disk, a magnetic hard disk drive, a solid-state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

`Computer memory' or 'memory' is an example of a computer-readable storage medium. Computer memory is any memory which is directly accessible to a computational system. `Computer storage' or 'storage' is a further example of a computer-readable storage medium. Computer storage is any non-volatile computer-readable storage medium. In some embodiments computer storage may also be computer memory or vice versa.

A `computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computational systems each comprising a processor or computational systems. The machine executable code or instructions may be executed by multiple computational systems or processors that may be within the same computational system, or which may even be distributed across multiple computational systems.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further understood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A `user interface' as used herein is an interface which allows a user or operator to interact with a computer or computer system. A `user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A `hardware interface' as used herein encompasses an interface which enables the computational system of a computer system to interact with and/or control an external computational system and/or apparatus. A hardware interface may allow a computational system to send control signals or instructions to an external computational system and/or apparatus. A hardware interface may also enable a computational system to exchange data with an external computational system and/or apparatus. Examples of a hardware interface include, but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or `display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

The term `machine learning' (ML) refers to a computer algorithm used to extract useful information from training datasets by building probabilistic models, which are referred to as machine learning modules or models, in an automated way. A machine learning module may also be referred to as a predictive model. Machine learning algorithms build a mathematical model based on sample data, known as 'training data', in order to make predictions or decisions without being explicitly programmed to perform the respective task. The machine learning module may be performed using a learning algorithm such as supervised or unsupervised learning. The machine learning module may be based on various techniques such as clustering, classification, linear regression, reinforcement, self-learning, support vector machines, neural networks, etc. A machine learning module may, e.g., be a data structure or program such as a neural network, in particular a convolutional neural network, a support vector machine, a decision tree, a Bayesian network etc. The machine learning module may be adapted, i.e., trained to predict an unmeasured value. The trained machine learning module may thus be enabled to predict the unmeasured value as output from other known values as input.

A machine learning module to be trained may, e.g., be an untrained machine learning module, a pre-trained machine learning module or a partially trained machine learning module. The machine learning module being trained may be an untrained machine learning module, which is trained from scratch. Alternatively, the machine learning module being trained may be a pre-trained or partially trained machine learning module. In general, it may not be necessary to start with an untrained machine learning module, e.g., in deep learning. For example, one may start with a pre-trained or partially trained machine learning module. The pre-trained or partially trained machine learning module may have been pre-trained or partially trained for the same or a similar task. Using a pre-trained or partially trained machine learning may, e.g., enable a faster training of the trained machine learning module to be trained, i.e., the training may converge faster. For example, transfer learning may be used for training a pre-trained or partially trained machine learning module. Transfer learning refers to a machine learning process, which rather than starting the learning process from scratch starts from patterns that have been previously learned, when solving a different problem. This way previous learnings may, e.g., be leveraged, avoiding to start from scratch. A pre-trained machine learning module is a machine learning module that was trained previously, e.g., on a large benchmark dataset to solve a problem similar to the one to be solved by the additional learning. In case of a pre-trained machine learning module a previous learning process has been completed successfully. A partially trained machine learning module is a machine learning module, which has been partially trained, i.e., the training process may not have been completed yet. A pre-trained or partially machine learning module may, e.g., be imported and trained to be used for the purposes disclosed herein.

### REFERENCE SIGNS LIST

- 100: medical system
- 102: medical imaging system
- 118: imaging phantom
- 119: light visor cross
- 120: patient table
- 122: keypoint element of table
- 123: keypoint element of phantom
- 124: material arranged on patient table
- 126: upper surface
- 128: reclining surface
- 130: vertical direction
- 132: horizontal direction
- 140: photosensor imaging system
- 144: photosensor
- 154: magnet
- 156: bore of magnet
- 157: imaging zone
- 158: imaging iso-center
- 160: magnetic field gradient coils
- 161: radio-frequency transmitter coil
- 162: magnetic field gradient coil power supply
- 163: transmitter
- 200: receiving first photosensor imaging data acquired using the photosensor imaging system
- 202: determining a first relative position of the imaging phantom relative to the patient table using the first photosensor imaging data
- 204: receiving second photosensor imaging data acquired using the photosensor imaging system
- 206: determining a fourth relative position of the first position of the patient table relative to the table reference position
- 208: determining a fifth relative position of the second position of the patient table relative to the first position of the patient table using the second photosensor imaging data
- 210: determining a second relative position of the second position of the patient table relative to the table reference position using the second photosensor imaging data
- 212: receiving medical imaging data of the imaging phantom arranged within the imaging zone, which is acquired by the medical imaging system
- 214: determining a third relative position of the imaging phantom relative to the imaging reference position of the medical imaging system using the medical imaging data
- 216: determining the relative position of the imaging reference position of the medical imaging system relative to the table reference position of the patient table
- 218: determining of one or more transformation parameters for a transformation between a first coordinate system and a second coordinate system using the determined relative position of the imaging reference position relative to a table reference position
- 220: defining the target position within the second coordinate system.
- 222: receiving third photosensor imaging data acquired using the photosensor imaging system
- 224: determining the third position of the patient table within the first coordinate system using the third photosensor imaging data
- 226: determining the movement required for moving the patient table from the determined third position to the target position using the one or more transformation parameters for the transformation between the first coordinate system and the second coordinate system
- 228: controlling an execution of the determined movement
- 240: providing first training datasets for training the keypoint elements detection module
- 242: training the keypoint detection module for detecting as output the labeled keypoint elements of the patient table of the second photosensor imaging training data of the first training datasets in response to receiving the first photosensor imaging training data of the respective first training datasets
- 244: training the keypoint detection module for further detecting as output the labeled keypoint elements of the imaging phantom of the second photosensor imaging training data of at least part of the first training datasets in response to receiving the first photosensor imaging training data of the respective first training datasets
- 250: providing second training datasets for training the phantom position detection module
- 252: training the phantom position detection module for determining as output the relative positions of the imaging phantom of the second training data of the second training datasets in response to receiving the first medical imaging training data of the respective second training datasets
- 260: receiving a plurality of photosensor imaging data acquired using the photosensor imaging system
- 262: detecting the keypoint elements within the plurality of photosensor imaging data using the keypoint detection module
- 264: checking that confidence values determined for a predefined number of the keypoint elements detected within photosensor imaging data of the plurality of photosensor imaging data exceed a predefined threshold
- 266: selecting the photosensor imaging data of the plurality of photosensor imaging data with confidence values of keypoint elements exceeding the predefined threshold
- 400: computational system
- 402: computational component
- 404: memory
- 406: hardware interface
- 408: user interface
- 410: machine readable instructions
- 412: keypoint detection module
- 414: photosensor imaging data
- 416: medical imaging data
- 418: phantom position detection module
- 420: specification of relative position of imaging reference position to table reference position
- 422: transformation parameter
- 424: training data
- 426: training data
- 500: 3D digital table model
- 504: label
- 520: random image element
- 550: 3D digital phantom model
- 554: label

## Claims

1. A method for calibrating a medical imaging system (102), the calibrating comprising by a computational system (400) a determining of a relative position of an imaging reference position of the medical imaging system (102) relative to a table reference position of a movable patient table (120) using a photosensor imaging system (140), the photosensor imaging system (140) being configured for a supervision of the medical imaging system (102) with the patient table (120),
the method comprising:
- receiving first photosensor imaging data (414) acquired using the photosensor imaging system (140), the first photosensor imaging data (414) comprising photosensor imaging data of the patient table (120) in an initial first position with an imaging phantom (118) arranged on the patient table (120);
- determining a first relative position of the imaging phantom (118) relative to the patient table (120) using the first photosensor imaging data (414);
- determining a second relative position of a second position of the patient table (120) relative to the table reference position, in the second position of the patient table (120) the imaging phantom (118) being arranged within an imaging zone (157) of the medical imaging system (102);
- receiving medical imaging data (416) of the imaging phantom (118) arranged within the imaging zone (157) acquired by the medical imaging system (102);
- determining a third relative position of the imaging phantom (118) relative to the imaging reference position of the medical imaging system (102) using the medical imaging data (416);
- determining the relative position of the imaging reference position of the medical imaging system (102) relative to the table reference position of the patient table (120) using the determined first, second, and third relative position.

2. The method of claim 1, the method further comprising receiving second photosensor imaging data (414) acquired using the photosensor imaging system (140), the second photosensor imaging data (414) comprising photosensor imaging data of the patient table (120) moved to the second position, the second photosensor imaging data (414) being used for the determining of the second relative position of the second position of the patient table (120) relative to the table reference position.

3. The method of claim 2, the initial first position differing from the table reference position, the method further comprising:
- determining a fourth relative position of the first position of the patient table (120) relative to the table reference position;
- determining a fifth relative position of the second position of the patient table (120) relative to the first position of the patient table (120) using the second photosensor imaging data (414);
- using the fourth and fifth relative position for determining the second relative position.

4. The method of any of the previous claims, the computational system (400) comprising a keypoint detection module (412) configured for detecting as output one or more keypoint elements (122) of the patient table (120) within the first photosensor imaging data (414) in response to receiving the first photosensor imaging data (414) as input, the keypoint detection module (412) being further configured for detecting as output one or more keypoint elements (123) of the imaging phantom (118) within the first photosensor imaging data (414) received as input,
the method further comprising:
- determining first keypoint elements (122) of the patient table (120) within the first photosensor imaging data (414) using the keypoint detection module (412);
- determining keypoint elements (123) of the imaging phantom (118) within the first photosensor imaging data (414) using the keypoint detection module (412);
- using the determined first keypoint elements (122) of the patient table (120) and the determined keypoint elements (123) of the imaging phantom (118) for the determining of the first relative position.

5. The method of claim 4, the method further comprising:
- determining the first position of the patient table (120) using the determined first keypoint elements (122);
- using the determined first position of the patient table (120) for the determining of the second relative position.

6. The method of any of claims 4 to 5, the keypoint detection module (412) being further configured for detecting as output one or more keypoint elements (122) of the patient table (120) within the second photosensor imaging data (414) in response to receiving the second photosensor imaging data (414) as input, the method further comprising:
- determining second keypoint elements (122) of the patient table (120) within the second photosensor imaging data (414) using the keypoint detection module (412);
- determining the second position of the patient table (120) using the determined second keypoint elements (122);
- using the determined second position of the patient table (120) for the determining of the second relative position.

7. The method of any of claims 4 to 6, the keypoint elements (122) of the patient table (120) being distributed over a surface of patient table (120), more particularly along a contour line of the patient table (120), and/or
the keypoint elements (123) of the imaging phantom (118) being located at a center of the imaging phantom (118) and/or the keypoint elements (123) being distributed over a surface of imaging phantom (118).

8. The method of any of claims 4 to 7, the method further comprising:
- providing first training datasets (424) for training the keypoint elements detection module, the first training datasets (424) comprising first photosensor imaging training data of the patient table (120) without keypoint elements (122) of the patient table (120) labeled within the first photosensor imaging training data and second photosensor imaging training data of the patient table (120) with the keypoint elements (122) of the patient table (120) labeled within the second photosensor imaging training data;
- training the keypoint detection module (412) for detecting as output the labeled keypoint elements (122) of the patient table (120) of the second photosensor imaging training data of the first training datasets (424) in response to receiving the first photosensor imaging training data of the respective first training datasets (424);
at least a part of the first training datasets (424) comprising second photosensor imaging training data of the imaging phantom (118) with keypoint elements (123) of the imaging phantom (118) labeled additionally within the respective photosensor imaging training data, the method further comprising:
- training the keypoint detection module (412) for further detecting as output the labeled keypoint elements (123) of the imaging phantom (118) of the second photosensor imaging training data of the at least part of the first training datasets (424) in response to receiving the first photosensor imaging training data of the respective first training datasets (424).

9. The method of any of the previous claims, the computational system (400) further comprising a phantom position detection module (418) configured for determining as output a relative position of the imaging phantom (118) relative to the imaging reference position of the medical imaging system (102) in response to receiving the medical imaging data (416) of the imaging phantom (118) as input, the phantom position detection module (418) being used for the determining of the third relative position of the imaging phantom (118).

10. The method of claim 9, the method further comprising:
- providing second training datasets (426) for training the phantom position detection module (418), the second training datasets (426) comprising first medical imaging training data of the imaging phantom (118) arranged within the imaging zone (157) of the medical imaging system (102) and second training data identifying relative positions of the imaging phantom (118) arranged within the imaging zone (157) relative to the imaging reference position of the medical imaging system (102);
- training the phantom position detection module (418) for determining as output the relative positions of the imaging phantom (118) of the second training data of the second training datasets (426) in response to receiving the first medical imaging training data of the respective second training datasets (426).

11. The method of any of the previous claims, the calibrating further comprising a determining of one or more transformation parameters (422) for a transformation between a first coordinate system and a second coordinate system using the determined relative position of the imaging reference position relative to the table reference position, the first coordinate system being a table coordinate system defined relative to the table reference position, the second coordinate system being an imaging coordinate system defined relative to the imaging reference position.

12. The method of claim 11, the method further comprising controlling a movement of the patient table (120) to a target position defined within the second coordinate system, the controlling comprising:
- receiving third photosensor imaging data (414) acquired using the photosensor imaging system (140), the third photosensor imaging data (414) comprising photosensor imaging data of the patient table (120) in an initial third position,
- determining the third position of the patient table (120) within the first coordinate system using the third photosensor imaging data (414),
- determining the movement required for moving the patient table (120) from the determined third position to the target position using the one or more transformation parameters (422) for the transformation between the first coordinate system and the second coordinate system,
- controlling an execution of the determined movement.

13. A computer program for calibrating a medical imaging system (102), the calibrating comprising a determining of a relative position of an imaging reference position of the medical imaging system (102) relative to a table reference position of a patient table (120) of the medical imaging system (102) using a photosensor imaging system (140), the photosensor (140) system being configured for a supervision of the medical imaging system (102),
the computer program comprising machine executable instructions (410) for an execution by a computational system (400), wherein execution of the machine executable instructions (410) causes the computational system (400) to perform the method of any of the previous claims.

14. A medical system (100) comprising a computational system (400) for calibrating a medical imaging system (102), the calibrating comprising a determining of a relative position of an imaging reference position of the medical imaging system (102) relative to a table reference position of a patient table (120) of the medical imaging system (102) using a photosensor imaging system (140), the photosensor imaging system (140) being configured for a supervision of the medical imaging system (102),
the computational system (400) comprising a memory (404) storing machine executable instructions (410), wherein execution of the machine executable instructions (410) causes the computational system (400) to perform the method of any of the previous claims 1 to 12.

15. The medical system (100) of claim 14, the medical system (100) further comprising one or more of the following:
the photosensor imaging system (140) configured for the supervision of the medical imaging system (102) with the patient table (120), the machine executable instructions being further configured for controlling the photosensor imaging system (140) to acquire the first and second photosensor imaging data;
the patient table (120);
the medical imaging system (102), the machine executable instructions (410) being further configured for controlling the medical imaging system (102) to acquire the medical imaging data (416) of the imaging phantom (118) arranged within the imaging zone (157) of the medical imaging system (102), the medical imaging system (102) being one of the following: a magnetic resonance imaging system, a computed tomography system, an X-ray imaging system;
the imaging phantom (118).
